# EUROPEAN PATENT SPECIFICATION

(11) **EP 1 697 356 B1**
(45) Date of publication and mention of the grant of the patent: **27.02.2008**
(21) Application number: 04801323.9
(22) Date of filing: 06.12.2004
(51) Int. Cl.: C07D 471/04, A61K 31/519, A61P 15/00, A61P 19/10, A61P 9/06, A61P 35/00

(54) **PYRIDO[2,3-D]PYRIMIDINE-2,4-DIAMINES AS PDE 2 INHIBITORS**
PYRIDO[2,3-D]PYRIMIDIN-2,4-DIAMINE ALS PDE-2-INHIBITOREN
PYRIDO [2,3-D]PYRIMIDINE-2,4-DIAMINES EN TANT QU'INHIBITEURS DE LA PDE 2

(30) Priority: 16.12.2003 US 529994 P
(43) Date of publication of application: 06.09.2006
(73) Proprietor: Pfizer Products Incorporated, Groton, CT 06340 (US)
(72) Inventor: BEYER, Thomas Arthur, Groton, CT 06340 (US); CHAMBERS, Robert James, Groton, CT 06340 (US); LAM, Kelvin, Cambridge, MA 02139 (US); LI, Mei, Groton, CT 06340 (US); MORRELL, Andrew Ian, Kent CT13 9NJ (GB); THOMPSON, David Duane, Groton, CT 06340 (US)
(74) Representative: Motion, Keith Robert
(86) International application number: PCT/IB2004/004013
(87) International publication number: WO 2005/061497

(56) References cited:
- WO-A-02/102315
- US-A- 5 547 954

## Description

### FIELD OF THE INVENTION

The invention relates to certain pyrido[2,3-d]pyrimidine-2,4-diamines useful as PDE 2 inhibitors; pharmaceutical formulations thereof; combinations thereof; and uses thereof.

### BACKGROUND OF THE INVENTION

The phosphodiesterase (PDE) family of enzymes regulates intracellular levels of secondary messenger cAMP or cGMP through hydrolytic control. Phosphodiesterase Type II (PDE 2) possesses a low affinity catalytic domain and an allosteric domain specific for cGMP. The low affinity catalytic site can hydrolyze both cAMP and cGMP with a lower apparent K_{M} for cGMP over cAMP. However, when cGMP binds to the allosteric site, the catalytic site undergoes a conformational change showing high affinity for cAMP. PDE 2 shows the highest expression in the brain, but is also found in many other tissues as well and, therefore, has a broad array of function and potential therapeutic utility (J. A. Beavo, et. al., Rev. Physio. Biochem. Pharm., 135, 67 (1999)). Examples of PDE 2 function and therapeutic potential are in neuronal development, learning, and memory (W. C. G. van Staveren, et. al., Brain Res., 888, 275 (2001) and J. O'Donnell, et. al., J. Pharm. Exp. Ther., 302, 249 (2002)); prolactin and aldosterone secretion (M. O. Velardez, et. al., Eur. J. Endo., 143, 279 (2000) and N. Gallo-Payet, et. al., Endo., 140, 3594 (1999)); bone cell differentiation, growth, and bone resorption (C. Allardt-Lamberg, et. al., Biochem. Pharm., 59, 1133 (2000) and S. Wakabayashi, et. al., J. Bone. Miner. Res., 17, 249 (2002)); immunological response (M. D. Houslay, et. al., Cell. Signal., 8, 97 (1996); vascular angiogenesis (T. Keravis, et. al., J. Vasc. Res., 37, 235 (2000); inflammatory cell transit (S. L. Wolda, et. al., J. Histochem. Cytochem., 47, 895 (1999); cardiac contraction (R. Fischmeister, et. al., J. Clin. Invest., 99, 2710 (1997), P. Donzeau-Gouge, et. al., J. Physiol., 533, 329 (2001), and D. J. Paterson, et. al., Card. Res., 52, 446 (2001)); platelet aggregation (R. J. Haslam, et. al., Biochem. J., 323, 371 (1997); female sexual arousal disorder (FSAD) (C.P. Wayman, et al., European Patent Application Publication Nos. EP 1 097 7707 and 1 0977 06); and hypoxic pulmonary vasoconstriction (J. Haynes, et. al., J. Pharm. Exp. Ther., 276, 752 (1996)). It has been shown that EHNA (erythro-9-(2-hydroxy-3-nonyl)adenine), a potent adenosine deaminase inhibitor, selectively inhibits PDE 2, however, the use of EHNA as a PDE 2 based therapeutic agent is limited due to low potency in inhibiting PDE 2, and high potency in inhibiting adenosine deaminase (R. Fischmeister, et. al., Mol. Pharm., 48, 121 (1995)).

It has now been found that certain pyrido[2,3-d]pyrimidine-2,4-diamine derivatives of formula (I) hereinbelow inhibit PDE 2 and, therefore, are useful in the treatment of physiological disorders mediated though the cAMP or cGMP cellular-signaling pathway.

U.S. Pat. Nos. 5,547,954 and 5,710,157 disclose certain 2,4-diamino-5,6-disubstituted- and 5,6,7-trisubstituted-5-deazapteridines, compositions thereof, and uses thereof in controlling insects in agricultural crops.

PDE 7 inhibitors are described in WO 02/102 315.

### SUMMARY OF THE INVENTION

The invention provides compounds of formula (1) 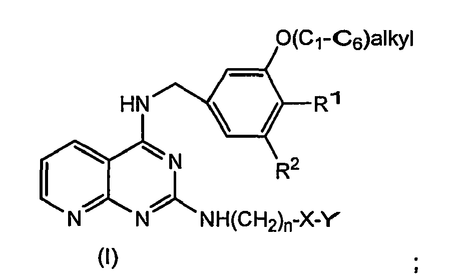 and the pharmaceutically acceptable salts of the compounds, wherein n, X, and Y are as defined hereinbelow; pharmaceutical compositions thereof; combinations thereof; and uses thereof.

### DETAILED DESCRIPTION OF THE INVENTION

The present invention provides compounds of formula (I) 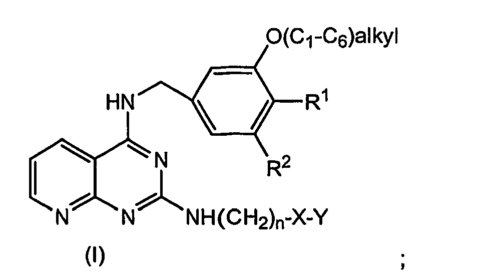 and the pharmaceutically acceptable salts of the compounds wherein:
R¹ and R² are hydrogen or methoxy, provided R¹ and R² are not both hydrogen or both methoxy;
n is 1, 2, 3, or 4;
X is a bond; O; S; C=O; -N(R)-, wherein R is hydrogen or -(C₁- C₃)alkyl; -C(OH)-; or -SO₂; and
Y is benzoxazolyl; benzothiazolyl; benzofurazanyl; benzofuranyl; benzothiadiazolyl; benzisoxazolyl; benzisothiazolyl; benzimidazolyl; pyridyl; isatinyl; oxindolyl; indazolyl; indolyl; phenyl; thienyl; or furanyl; wherein Y is optionally substituted independently with from one to three halogen; trifluoromethyl; methoxy; -C(=O)CH₃; cyano; -C(CH₃)₂OH; -CH(CH₃)OH; -CH(CF₃)OH; -C(C=O)CF₃; -SO₂NH₂; -C(=O)OCH₃; -CH₂COOH; 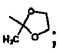 thiazolyl; or oxadiazolyl.

A generally preferred subgroup of the compounds of formula (I) comprises those compounds wherein X is a bond and Y is benzofurazanyl; thienyl; pyridyl; or phenyl, wherein phenyl is optionally substituted independently with one or two halogen; trifluoromethyl; methoxy; -C(=O)CH₃; cyano; -C(CH₃)₂OH; -CH(CH₃)OH; -CH(CF₃)OH; -C(C=O)CF₃; -SO₂NH₂; -C(=O)OCH₃; -CH₂COOH; thiazolyl; or oxadiazolyl.

An especially preferred subgroup of the compounds of formula (I) comprises those compounds wherein X is a bond, n is 2 or 3, and Y is thienyl; pyridyl; or phenyl, wherein phenyl is optionally substituted independently with one or two methoxy; halogen; -C(CH₃)₂OH; CH(CF₃)OH; or -C(C=O)CF₃.

A cyclic group may be bonded to another group in more than one way. If no particular bonding arrangement is specified, then all possible arrangements are intended. For example, the term "pyridyl" includes 2-, 3-, or 4-pyridyl, and the term "thienyl" includes 2- or 3-thienyl.

The compounds and intermediates of the present invention may be named according to either the IUPAC (International Union for Pure and Applied Chemistry) or CAS (Chemical Abstracts Service, Columbus, OH) nomenclature systems.

The carbon atom content of the various hydrocarbon-containing moieties may be indicated by a prefix designating the minimum and maximum number of carbon atoms in the moiety, i.e., the prefix "-(Cₐ-C_{b})alkyl" indicates an alkyl moiety of the integer "a" to "b" carbon atoms, inclusive.

The term "alkyl" denotes straight or branched, monovalent chains of carbon atoms. Examples of alkyl groups include methyl, ethyl, propyl, isopropyl, butyl, isobutyl, and the like.

The term "halogen" represents chloro, fluoro, bromo, and iodo.

The term "mammal" means animals including, for example, dogs, cats, cows, sheep, horses, and humans. Preferred mammals include humans of either gender.

The term "salts" refers to organic and inorganic salts of a compound of formula (I), or a prodrug thereof. These salts can be prepared *in situ* during the final isolation and purification of a compound, or by separately reacting a compound of formula (I), or a prodrug thereof, with a suitable organic or inorganic acid or base and isolating the salt thus formed. Representative salts include the hydrobromide, hydrochloride, sulfate, bisulfate, nitrate, acetate, oxalate, besylate, palmitate, stearate, laurate, borate, benzoate, lactate, phosphate, tosylate, citrate, maleate, fumarate, succinate, tartrate, naphthylate, mesylate, glucoheptonate, lactobionate, and laurylsulphonate salts, and the like. These may also include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. For additional examples see, for example, Berge, et al., J. Pharm. Sci., 66, 1-19 (1977).

The term "substituted" means that a hydrogen atom on a molecule has been replaced with a different atom or molecule. The atom or molecule replacing the hydrogen atom is denoted as a "substituent."

As used herein, the term "therapeutically effective amount" means an amount of a compound that is capable of treating a described pathological condition.

The terms "treat", "treatment", and "treating" include preventative (e.g., prophylactic) and palliative (e.g., healing or curative) treatment, or the act of providing preventative or palliative treatment.

The compounds of formula (I) may contain asymmetric or chiral centers and, therefore, exist in different stereoisomeric forms. It is intended that all stereoisomeric forms of the compounds and prodrugs of formula (I) as well as mixtures thereof, including racemic mixtures, form part of the present invention. In addition, the present invention embraces all geometric and positional isomers. For example, if a compound or prodrug of formula (I) incorporates a double bond(s), both the *cis*- and *trans-* forms, as well as mixtures thereof, are embraced within the scope of the invention.

Diastereomeric mixtures can be separated into their individual diastereomers on the basis of their physical chemical differences by methods well-known to those of ordinary skill in the art, such as by chromatography and/or fractional crystallization. Enantiomers can be separated by converting the enantiomeric mixture into a diasteriomeric mixture by reaction with an appropriate optically active compound (e.g., alcohol), separating the diastereomers and converting (e.g., hydrolyzing) the individual diastereomers to the corresponding pure enantiomers.

The compounds of the compounds of formula (I) may exist in unsolvated as well as solvated forms with pharmaceutically acceptable solvents, such as water, ethanol, and the like, and it is intended that the invention embrace both solvated and unsolvated forms.

It is also possible that the compounds of formula (I) may exist as tautomeric isomers in equilibrium, and all such forms are embraced within the scope of the invention.

The present invention also embraces isotopically-labeled compounds of formula (I), which are identical to those recited herein, but for the fact that one or more atoms are replaced by an atom having an atomic mass or mass number different from the atomic mass or mass number usually found in nature. Examples of isotopes that can be incorporated into compounds of formula (I) include isotopes of hydrogen, carbon, nitrogen, oxygen, phosphorus, fluorine, and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. The compounds of formula (I), and the pharmaceutically acceptable salts of the compounds, that contain the aforementioned isotopes and/or other isotopes of the other atoms are intended to be within the scope of the instant invention.

Certain isotopically-labeled compounds of formula (I), for example those compounds into which radioactive isotopes such as ³H and ¹⁴C are incorporated, are useful in compound and/or substrate tissue distribution assays. Tritiated, i.e., ³H, and carbon-14, i.e., ¹⁴C, isotopes are particularly preferred for their relative ease of preparation and facile detection. Furthermore, substitution with heavier isotopes such as deuterium, i.e., ²H, may afford certain therapeutic advantages resulting from greater metabolic stability, for example, increased *in vivo* half-life, or reduced dosage requirements and, hence, may be preferred in some circumstances. The isotopically-labeled compounds of formula (I) can generally be prepared by carrying out procedures analogous to those disclosed in the Schemes and/or Examples set forth hereinbelow, by substituting an isotopically-labeled reagent for a non-isotopically-labeled reagent.

In another aspect, the invention provides the use of a compound of formula (I) in the manufacture of a medicament for treating PDE 2-mediated conditions, diseases, or symptoms in a mammal in need of such treatment which methods comprise administering to the mammal a therapeutically effective amount of a compound of formula (I), a prodrug thereof, or a pharmaceutically acceptable salt of the compound or prodrug; or a pharmaceutical composition comprising a compound of formula (I), a prodrug thereof, or a pharmaceutically acceptable salt of the compound or prodrug, and a pharmaceutically acceptable vehicle, carrier, or diluent.

Preferred conditions, diseases, or symptoms treatable according to the present methods include osteoporosis, pulmonary hypertension, female sexual arousal disorder, diminished memory or cognition, platelet aggregation, vascular angiogenesis, dementia, cancer, arrhythmia, thrombosis, bone fracture and/or defect, delayed or non-union fracture, spinal fusion, bone in-growth, cranial facial reconstruction, or hypoxia. An especially preferred condition is bone fracture and/or defect.

In another aspect, the invention provides the use of a compound of formula (I) in the manufacture of a medicament for inhibiting PDE 2 activity in a mammal in need of such inhibition which methods comprise administering to the mammal a PDE 2-inhibiting amount of a compound of formula (I), or a pharmaceutically acceptable salt of the compound; or a pharmaceutical composition comprising a compound of formula (I), or a pharmaceutically acceptable salt of the compound, and a pharmaceutically acceptable vehicle, carrier, or diluent.

The compounds of formula (I), and the pharmaceutically acceptable salts of the compounds and prodrugs, may be administered to a mammal at dosage levels in the range of from about 0.001 mg to about 200 mg per day. For a normal adult human having a body mass of about 70 kg, a dosage in the range of from about 0.01 mg to about 100 mg per kg body mass is typically preferred, however, some variability in the general dosage range may be required depending upon the age and mass of the subject being treated, the intended route of administration, the particular compound being administered, and the like. The determination of dosage ranges and optimal dosages for a particular mammalian subject is within the ability of one of ordinary skill in the art having benefit of the instant disclosure.

In yet another aspect, the invention provides pharmaceutical compositions comprising a combination of a PDE 2 inhibitor according to formula (I), an EP₂ selective agonist; and a pharmaceutically acceptable vehicle, carrier, or diluent; and methods of treating osteoporosis, pulmonary hypertension, female sexual arousal disorder, diminished memory or cognition, platelet aggregation, vascular angiogenesis, dementia, cancer, arrhythmia, thrombosis, bone fracture and/or defect, delayed or non-union fracture, spinal fusion, bone in-growth, cranial facial reconstruction, or hypoxia using such compositions. An especially preferred condition is bone fracture and/or defect.

In yet another aspect, the invention provides the use of a compound of formula (I) in the manufacture of a medicament for treating bone fracture and/or defect in a mammal in need of such treatment which methods comprise administering to the mammal a therapeutically effective amount of a PDE 2 inhibitor, or a pharmaceutically acceptable salt of said inhibitor.

Any PDE 2 inhibitor, including the compounds of formula (I) herein, can be employed in the methods and combinations of the invention. Examples of known PDE 2 inhibitors comprise EHNA, 6-(3,4-dimethoxy-benzyl)-1-[(1-hydroxy-ethyl)-4-phenyl-butyl]-3-methyl-1,5-dihydro-pyrazolo[3,4-d]pyrimidine-4-one (BAY-60-7550; U.S. Pat. No. 6,174,884), and 9-(1-acetyl-4-phenyl-butyl)-2-(3,4-dimethoxy-benzyl)-1,9-dihydropurin-6-one (U.S. Pat. No. 5,861,396). Additional examples of PDE 2 inhibitors are disclosed in U.S. Pat. Nos. 5,861,396; 5,401,774; 6,458,796; and 6,555,547; and in PCT International Application Publication No. 98/32755.

Any EP₂ selective receptor agonist can be employed in the combination aspects of the present invention, however, a generally preferred class of EP₂ selective receptor agonists, disclosed in commonly-assigned U.S. Patent Number 6,498,172, comprises compounds of Formula AA 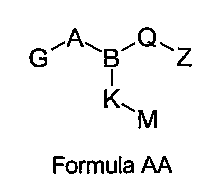 prodrugs thereof, and the pharmaceutically acceptable salts thereof, wherein G, A, B, K, M, Q, and Z are as defined therein.

Generally preferred compounds of Formula AA are (3-(((pyridine-3-sulfonyl)-(4-pyrimidin-5-yl-benzyl)-amino)-methyl)-phenyl)-acetic acid; (3-(((5-phenyl-furan-2-ylmethyl)-(pyridine-3-sulfonyl)-aminormethyl)-phenyl)-acetic acid; (3-(((pyridine-3-sulfonyl)-(4-pyrimidin-2-yl-benzyl)-amino)-methyl)-phenyl)-acetic acid; (3-(((pyridine-3-sulfonyl)-(4-thiazol-2-yl-benzyl)-amino)-methyl)-phenyl)-acetic acid; (3-(((4-pyrazin-2-yl-benzyl)-(pyridine-3-sulfonylfamino)-methyl)-phenyl)-acetic acid; (3-(((4-cyclohexylbenzyl)-(pyridine-3-sulfonylhamino)-methyl)-phenoxyhacetic acid; (3-(((pyridine-3-sulfonyl)-(4-pyridin-2-yl-benzyl)-amino)-methyl)-phenoxy)-acetic acid; (3-(((pyridine-3-sulfonyl)-(4- pyridin-3-yl-benzyl)-amino)-methyl)-phenoxy)-acetic acid; (3-(((pyridine-3-sulfonyl)-(4-pyridin-4-yl-benzyl)-amino)-methyl)-phenoxy)-acetic acid; (3-(((pyridine-3-sulfonyl)-(4-thiazol-2-yl-benzyl)amino)methyl)-phenoxy)-acetic acid; 5-(3-((pyridine-3-sulfonyl)-(4-thiazol-2-yl-benzyl)-amino)-propyl)-thiophene-2-carboxylic acid; (3-(((2,3-dihydro-benzo[1,4]dioxin-6-ylmethyl)-(pyridine-3-sulfonyl)-aminormethyl)-phenyl)-acetic acid; and (3-((benzofuran-2-ylmethyl-(pyridine-3-sulfonyl)-amino)-methyl)-phenyl)-acetic acid; (3-(((4-butyl-benzyl)-(pyridine-3-sulfonyl)-amino)-methyl)-phenyl)-acetic acid; (3-((benzenesulfonyl-(4-butyl-benzyl)-amino)-methyl)-phenyl)-acetic acid; (3-(((4-butyl-benzyl)-(1-methyl-1H-imidazole-4-sulfonyl)-amino)-methyl)-phenyl)-acetic acid; and (3-(((4-dimethylamino-benzyl)-(pyridine-3-sulfonyl)-amino)-methyl)-phenyl)-acetic acid; (3-(((4-dimethylamino-benzyl)-(pyridine-3-sulfonyl)-amino)-methyl)-phenoxy)-acetic acid and (3-(((4-*tert*-butyl-benzyl)-(pyridine-3-sulfonyl)-amino)-methyl)-phenoxy)-acetic acid; *trans*-(3-(((3-(3,5-dichloro-phenyl)-allyl)-(pyridine-3-sulfonyl)-amino)-methyl)-phenyl)-acetic acid; (3-(((2-(3,5-dichlorophenoxy)-ethyl)-(pyridine-3-sulfonyl)-amino)-methyl)-phenoxy)-acetic acid; prodrugs thereof, and pharmaceutically acceptable salts of the compounds and the prodrugs.

An especially preferred compound of Formula AA is (3-(((4-*tert*-butyl-benzyl)-(pyridine-3-sulfonyl)-amino)-methyl)-phenoxy)-acetic acid, a prodrug thereof, or a pharmaceutically acceptable salt of the compound or prodrug. A particularly preferred salt is the sodium salt.

Another generally preferred class of EP₂ selective receptor agonists useful in the combination aspects of the invention comprises the compounds, prodrugs, and pharmaceutically-acceptable salts of Formula BB below, which are disclosed in commonly-assigned U.S. Pat. No. 6,288,120 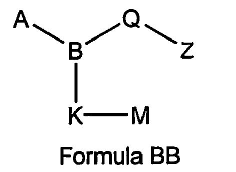 wherein A, B, K, M, Q, and Z are as defined therein.

Generally preferred compounds of Formula BB are 7-[(2'-hydroxymethylbiphenyl-4-ylmethyl)-methanesulfonyl-amino]-heptanoic acid; 7-{[4-(3-hydroxymethylthiophen-2-yl)-benzyl]-methanesulfonyl-amino}-heptanoic acid; 7-[(2'-chloro-biphenyl-4-ylmethyl)-methanesulfonyl-amino]-heptanoic acid; 7{[4-(1-hydroxy-hexyl)-benzyl]-methanesulfonyl-amino]-heptanoic acid; 7-[(4-butyl-benzyl)-methanesulfonyl-amino]-heptanoic acid; 7-{[5-(1-hydroxy-hexyl)-thiophen-2-ylmethyl]-methanesulfonyl-amino}-heptanoic acid; (3-{[(4-butyl-benzyl)-methanesulfonyl-amino]-methyl}phenyl)acetic acid; 7-{[3-(3-Chloro-phenyl)-propyl]-methanesulfonyl-amino}-heptanoic acid; 7-{[3-(3,5-Dichloro-phenyl)-propyl]-methanesulfonyl-aminol}-heptanoic acid; 5-(3-{[3-(3-chloro-phenyl)-propyl]-methanesulfonyl-amino}-propyl)-thiophene-2-carboxylic acid; 7-{[2-(3,5-dichloro-phenoxy)-ethyl]-methanesulfonyl-amino}-heptanoic acid; 5-(3-{[2-(3,5-dichloro-phenoxy)-ethyl]-methanesulfonyl-amino}-propyl)-thiophene-2-carboxylic acid; N-[2-(3,5-dichloro-phenoxy)-ethyl]-N-[6-(1H-tetrazol-5-yl)-hexyl]-methanesulfonamide; *trans*-(4-{[3-(3,5-dichloro-phenyl)-allyl]-methanesulfonylamino}-butoxy)-acetic acid; *trans*-N-[3-(3,5-dichloro-phenyl)-allyl]-N-[6-(1H-tetrazolyl-5-yl)-hexyl]-methanesulfonamide; *trans*-5-(3-{[3-(3,5-dichloro-phenyl)-allyl]-methanesulfonyl-amino}-propyl)-thiophene-2-carboxylic acid; *trans*-[3-({[3-(3,5-dichloro-phenyl)-allyl]-methanesulfonyl-amino}-methyl)-phenyl]-acetic acid; the prodrugs thereof, and the pharmaceutically acceptable salts of the compounds and prodrugs.

An especially preferred compound of Formula BB is 7-[(4-butyl-benzyl)-methanesulfonyl-amino]-heptanoic acid, a prodrug thereof, or a pharmaceutically acceptable salt of the compound or prodrug. A preferred salt is the monosodium salt.

Other EP₂ selective receptor agonists useful in the combination aspects of the present invention comprise the prostaglandin receptor agonists disclosed in U.S. Pat. Nos. 6,531,485; 6,376,533; 6,124,314; 5,877,211; 5,716,835; 5,698,598; and 5,462,968; U.S. Published Pat. Appl'n. No. 2002/187961; N. Duckworth, et al., Journal of Endocrinology, 172 (2), 263-269 (2002); K. Tani, et al., Synlett, 2, 239-242 (2002); K. Tani, et al., Bioorganic & Medicinal Chemistry, 10 (4), 1107-1114 (2002); K. Tani, et al., Bioorganic & Medicinal Chemistry, 10 (4), 1093-1106 (2002); J. Michelet, et al., EP 1 175 891 A1; K. Tani, et al., Bioorganic & Medicinal Chemistry Letters, 11 (15), 2025-2028 (2001); J. Y. Crider, et al., International Journal of Environmental Studies, 58 (1), 35-46 (2000); J. Y. Crider, et al., Journal of Ocular Pharmacology and Therapeutics, 17 (1), 35-46 (2001); D. F. Woodward, et al., Journal of Ocular Pharmacology and Therapeutics, 11 (3), 447-54 (1995); A. T. Nials, et al., Cardiovascular Drug Reviews, 11 (2), 165-79 (1993); and D. F. Woodward, et al., Prostaglandins, 46 (4), 371-83 (1993).

In the combination aspects of the invention, the EP₂ selective receptor agonists may be administered to mammals at dosage levels ranging from about 0.001 mg/kg to about 100 mg/kg body mass per day. For a normal adult human having a body mass of about 70 kg, a dosage in the range of from about 0.01 mg/kg to about 50 mg/kg body mass is typically preferred, however, some variability in the general dosage range may be required depending upon the age and mass of the subject being treated, the intended route of administration, the particular compound being administered, and the like. The determination of combination dosage ranges and optimal dosages for a particular mammalian subject is within the ability of one of ordinary skill in the art having benefit of the instant disclosure.

Pharmaceutical compositions suitable for parenteral injection may comprise pharmaceutically acceptable sterile aqueous or nonaqueous solutions, dispersions, suspensions, or emulsions, and sterile powders for extemporaneous reconstitution into sterile injectable solutions or dispersions. Examples of suitable aqueous and nonaqueous carriers, vehicles, and diluents include water, ethanol, polyols (such as propylene glycol, polyethylene glycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions, and by the use of surfactants.

The pharmaceutical compositions of the invention may further comprise adjuvants, such as preserving, wetting, emulsifying, and dispersing agents. Prevention of microorganism contamination of the instant compositions can be accomplished with various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions may be effected by the use of agents capable of delaying absorption, for example, aluminum monostearate and gelatin.

Solid dosage forms for oral administration include capsules, tablets, powders, and granules. In such solid dosage forms, the active compound is admixed with at least one inert conventional pharmaceutical excipient (or carrier) such as sodium citrate or dicalcium phosphate, or (a) fillers or extenders, as for example, starches, lactose, sucrose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid certain complex silicates, and sodium carbonate; (e) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and/or (i) lubricants, as for example, talc, calcium stearate, magnesium stearate, solid polyethylene glycols, sodium lauryl sulfate, or mixtures thereof. In the case of capsules and tablets, the dosage forms may further comprise buffering agents.

Solid compositions of a similar type may also be employed as fillers in soft or hard filled gelatin capsules using such excipients as lactose or milk sugar, as well as high molecular weight polyethylene glycols, and the like.

Solid dosage forms such as tablets, dragees, capsules, and granules can be prepared with coatings and shells, such as enteric coatings and others well-known to one of ordinary skill in the art. They may also comprise opacifying agents, and can also be of such composition that they release the active compound(s) in a delayed, sustained, or controlled manner. Examples of embedding compositions that can be employed are polymeric substances and waxes. The active compound(s) can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil, and sesame seed oil, glycerol, tetrahydrofurfuryl alcohol, polyethylene glycols and fatty acid esters of sorbitan, or mixtures of these substances, and the like.

Besides such inert diluents, the pharmaceutical composition can also include adjuvants, such as wetting agents, emulsifying and suspending agents, sweetening, flavoring, and perfuming agents.

Suspensions, in addition to the active compound(s), may further comprise suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of the aforementioned substances, and the like.

Compositions for rectal or vaginal administration preferably comprise suppositories, which can be prepared by mixing an active compound(s) with suitable non-irritating excipients or carriers such as cocoa butter, polyethylene glycol or a suppository wax, which are solid at ordinary room temperature, but liquid at body temperature, and therefore, melt in the rectum or vaginal cavity thereby releasing the active component.

Dosage forms for topical administration may comprise ointments, powders, sprays and inhalants. The active agent(s) are admixed under sterile condition with a pharmaceutically acceptable carrier, vehicle, or diluent, and any preservatives, buffers, or propellants that may be required.

A generally preferred composition for administering PDE 2 inhibitors, including the compounds of formula (I), as well as the combinations comprising PDE 2 inhibitors and EP₂ selective receptor agonists in the treatment of bone fractures, comprises an injectable, flowable composition that provides sustained release at a local site of injection by forming a biodegradable solid or gel depot, matrix, or implant. An example of such an administration system comprises a slow-release, biodegradable polymer-based delivery system. See, for example, U.S. Published Pat. Appl'n. No. 2003-0104031 A1.

Such a polymer-based delivery system generally comprises a therapeutically useful agent(s), dissolved or dispersed in a flowable, biodegradable, thermoplastic polymer solution or dispersion in an organic solvent. Upon injection of the composition, the organic solvent diffuses away from the injection site, causing the polymer to precipitate or gel, thereby entrapping the agent(s) in a sustained-release depot. The agent(s) is subsequently released by diffusion from, and erosion of, the polymeric matrix. The matrix slowly erodes by hydrolysis and eventually disappears from the site of administration. The molecular weight and concentration of the polymer can control the *in vivo* release of the agent(s) as well as the degradation rate of the matrix.

The polymer-based delivery system provides sustained release of an active agent(s) *in vivo* for a sustained period of time with minimum or reduced burst in a patient in need thereof. A large burst of agent(s) would result in poor local toleration due to local effects (e.g., irritation) and would minimize the amount of agent(s) available for efficacy. The advantage this administration method offers is that it minimizes or reduces the initial burst, but still delivers the agent(s) at efficacious levels for sustained periods of time upon a single local injection.

The polymeric system is prepared by contacting the flowable composition with a gelation medium to coagulate or gel the composition into a solid, microporous polymeric matrix, or a gel polymeric matrix. The flowable composition contains a thermoplastic polymer or copolymer in combination with a suitable solvent. The polymers or copolymers, which form the body of the matrix, are substantially insoluble, preferably essentially completely insoluble, in water and bodily fluids. The insolubility of the matrix body enables it to function as a single site for the controlled release of the agent(s). The polymers or copolymers are also biocompatible and biodegradable and/or bioerodible within the body of an animal, e.g., mammal. The biodegradation enables the patient to metabolize and excrete the polymeric matrix such that there is no need for surgical removal. Because the flowable composition and polymer system are biocompatible, the insertion process and the presence of the polymer system within the body do not cause substantial tissue irritation or necrosis at the implant site. The composition of the present invention is administered as a flowable composition directly into bodily tissues.

Suitable thermoplastic polymers for incorporation into the solid matrix of the controlled-release system are solids, pharmaceutically compatible and biodegradable by cellular action and/or by the action of bodily fluids. Examples of appropriate thermoplastic polymers include polyesters of diols and dicarboxylic acids or of hydroxycarboxylic acids, such as polylactides, polyglycolides, and copolymers thereof. More preferably the polymer comprises the copolymer, polylactic-co-glycolic acid (abbreviated PLGH) which, upon hydrolysis, produces lactic and glycolic acid. The burst of release of this copolymer can be minimized further by the addition of polyethylene glycol (PEG) to form the PEG end-capped PLGH.

Preferred materials comprise polylactides, polyglycolides, and copolymers thereof. These polymers can be used to advantage in the polymer system in part because they show excellent biocompatibility. They produce little, if any, tissue irritation, inflammation, necrosis, or toxicity. In the presence of water, the polymers produce lactic and glycolic acid, respectively, which are readily metabolized. The polylactides can also incorporate glycolide monomer to enhance the resulting polymeric degradation. These polymers are also preferred because they effectively control the rate of release of agent(s) from the polymeric system, and because they result in the local retention of the agent(s) at the site of the site of administration.

The solubility or miscibility of a thermoplastic polymer in the organic solvent of the composition will vary according to factors such as crystallinity, hydrophilicity, capacity for hydrogen bonding, and the molecular weight of the polymer. Consequently, the molecular weight and the concentration of the polymer in the solvent are adjusted to achieve desired miscibility, as well as a desired release rate for the incorporated agent(s).

The flowable composition of thermoplastic polymer, solvent, and the agent(s) comprises a stable flowable substance. A homogenous solution of the agent(s) in an organic solvent preferably results. The thermoplastic polymer is substantially soluble in the organic solvent. Upon placement of the flowable composition into the body, the solvent dissipates and the polymer solidifies or gels to form the polymeric system having the agent(s) within a solid or gel polymeric matrix.

For certain preferred polymers, the molecular weight of the polymer or copolymer is adjusted to be within a range of about 0.2 to about 0.4 inherent viscosity (I. V. in deciliters/g) for effective sustained release of the bone growth promoting compound. The typical rate of release of the incorporated agent(s) occurs at an I.V. of about 0.2 (about 8,000 to about 16,000 molecular weight) or about 0.3 (about 23,000 to about 45,000 molecular weight), but can vary depending on the particular components of the composition. For most systems, it is preferred to adjust the molecular weight of the polymer to about 0.2 I.V. for an effective sustained release of the agent(s).

For a poly(DL-lactide) or a lactide-co-glycolide polymer system, the desired molecular weight range is about 0.2 to about 0.4 I.V., with an I.V. of about 0.2 being preferred. The molecular weight of a polymer can be modified by conventional methods.

Especially preferred, commercially available thermoplastic polymers comprise the following: PLGH copolymer with 1:1 ratio of lactic and glycolic acid with an inherent viscosity of about 0.2 dl/g (commercially available from Boehringer Ingelheim as Copolymer RESOMER^{®} RG 502 H) (about 12,000 molecular weight); PLGH copolymer with 1:1 ratio of lactic and glycolic acid with an inherent viscosity of about 0.3 dl/g (commercially available from Boehringer Ingelheim as Copolymer RESOMER^{®} RG 503 H)(about 37,000 molecular weight); PLGH copolymer with 1:1 ratio of lactic and glycolic acid with an inherent viscosity of about 0.4 dl/g (commercially available from Boehringer Ingelheim as Copolymer RESOMER^{®} RG 504 H) (about 47,000 molecular weight); and polyethylene glycol (PEG) end-capped PLGH copolymer with 1:1 ratio of lactic and glycolic acid with an inherent viscosity of about 0.79 dl/g (commercially available from Boehringer Ingelheim as PLG-PEG) (about 52,000 molecular weight).

The solvents employed in the thermoplastic compositions are preferably pharmaceutically acceptable, biocompatible, and will dissipate into bodily fluid *in situ* such that they may be classed as having a solubility in water ranging from highly soluble to insoluble. Preferably, they cause relatively little, if any, tissue irritation or necrosis at the site of the injection and implantation. Preferably, the solvent will have at least a minimal degree of water solubility. When the organic solvent is water-insoluble or is minimally soluble in water, the solvent will slowly disperse from the flowable polymeric composition. The result will be an implant that, during the course of its life, may contain varying amounts of residual solvent. Preferably, the organic solvent has a moderate to high degree of water solubility so that it will facilely disperse from the polymeric composition into bodily fluids. Most preferably, the solvent disperses rapidly from the polymeric composition so as to quickly form a solid implant. Concomitant with the dispersion of solvent, the thermoplastic polymer coagulates or gels into the solid polymeric system. Preferably, as the thermoplastic polymer coagulates, the solvent dispersion causes pore formation within the polymer system. As a result, the flowable composition containing thermoplastic polymer, solvent, and agent(s) will form a porous solid polymer system. Also, when the solvent is slightly water-soluble, or is water-insoluble, the solvent dispersion may result in the formation of a solid porous implant, or if some solvent remains with the implant, the result may be formation of a gel implant having few or no pores.

Suitable solvents include those liquid organic compounds meeting the foregoing criteria. A generally preferred solvent comprises N-methyl-2-pyrrolidone (NMP).

The solvents for the thermoplastic polymer flowable compositions are chosen for compatibility and appropriate solubility of the polymer and solvent. Lower molecular weight thermoplastic polymers will normally dissolve more readily in the solvents than high molecular weight polymers. As a result, the concentration of a thermoplastic polymer dissolved in the various solvents differs depending upon type of polymer and its molecular weight. Conversely, the higher molecular weight thermoplastic polymers will tend to coagulate, gel or solidify faster than the very low molecular weight thermoplastic polymers. Moreover, the higher molecular weight polymers tend to give higher solution viscosities than the low molecular weight materials. Thus, for advantageous injection efficiency, in addition to advantageous release rate, the molecular weight and the concentration of the polymer in the solvent are controlled.

Upon formation of the polymer system from the flowable composition, the agent(s) becomes incorporated into the polymeric matrix. After insertion of the flowable composition to form the polymeric system, the agent(s) is released from the matrix into the adjacent tissues or fluids by diffusion and degradation mechanisms. Manipulation of these mechanisms also can influence release of the agent(s) into the surroundings at a controlled rate. For example, the polymeric matrix can be formulated to degrade after an effective and/or substantial amount of the agent(s) is released from the matrix. Thus, release of the agent(s) from the matrix can be varied by, for example, the solubility of the agent(s) in water, the distribution of the bone growth-promoting compound within the matrix, or the size, shape, porosity, solubility, and biodegradability of the polymer matrix, among other factors. The release of the agent(s) from the matrix is controlled relative to its inherent rate by varying the polymer molecular weight to provide a desired duration and rate of release.

For example, a preferred dosage form of the agent(s) comprises a lyophile to be reconstituted with a solution of PLGH in NMP before administration. The dosage form, consisting of the lyophilized compound in one syringe (syringe A) and a solution of PLGH in NMP in a second syringe (syringe B), is known as the A/B reconstitution system. The contents of both syringes are mixed together immediately prior to dose delivery at or near site. After reconstitution, the contents are transferred into a graduated dosing syringe for delivery. The administered dosage forms will be a solution and will result in the dispersion of the compound with PLGH in NMP at desired strengths of, for example, 5 and 50 mgA/ml (mgA/ml refers to the free acid equivalent of the sodium salt form of the agent(s)). The dosage form is a parenteral (e.g., subcutaneous, intramuscular, or intramedullary) sustained-release injection for local administration. This compound in a slow-release polymer matrix (depot injection) is designed for administration at or near a site, and is not intended for intravenous administration. To provide adequate shelf-life stability for the dosage form, a two-syringe system (A/B), as described above, may be used, preferably with the sodium salt form of the compound. A uniphase formulation, preferably with the free acid form of the compound, is a preferred alternative formulation. Based on the agent(s) and polymer stability, sterile filtration of the agent(s) and irradiation of the polymer solution may be preferred for manufacturing a stable sterile product. In one embodiment, the dosage form can be manufactured and shipped as separate aluminum pouches containing syringes filled with the lyophile form of the agent(s) in one pouch and the polymer solution in the other pouch. Delivery containers, systems, and methods for the lyophilization of bone growth promoting compounds are described in published PCT International Patent Application Publication No. WO 01/73363. Other methods of administration include local administration by injection to a particular site or delivery by a catheter to a site. Additional examples can be found in U.S. Provisional Application No. 60/335,156, filed November 30, 2001.

The teachings of all U.S. Pat. Nos. disclosed herein are incorporated by reference in their entirety.

The compounds of formula (I), and the pharmaceutically acceptable salts of the compounds, may be prepared according to the exemplary synthetic routes disclosed in the Schemes and Examples hereinbelow, as well as by other conventional organic preparative methods known, or apparent in light of the instant disclosure, to one of ordinary skill in the relevant art. The methods disclosed in the instant Schemes are intended for purposes of exemplifying the instant invention and are not to be construed in any manner as limitations thereon.

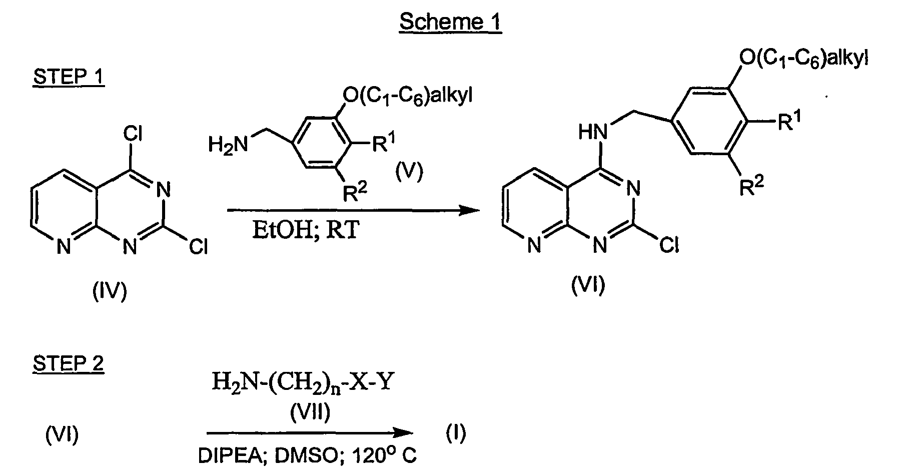

In Scheme 1, Step 1, 2,4-dichloro-pyrido[2,3-d]pyrimidine (lV) is reacted with an appropriately-substituted benzylamine (V) in the presence of a tri-substituted amine base, such as triethylamine (TEA) or diisopropylethylamine (DIPEA), or an aromatic base, such as pyridine. The reaction is typically effected in a polar alcoholic solvent, such as methanol (MeOH), ethanol (EtOH), or isopropanol (IPA), at a temperature ranging from about 0° C to about 100° C. Preferably, the reaction is effected in the presence of DIPEA in ethanol at about room temperature (RT). In Step 2, the resulting condensation product (VI) is then reacted with an appropriately-substituted amine (VII) in the presence of a tri-substituted amine base, such as TEA or DIPEA, or an aromatic base, such as pyridine, to afford compound (I). The reaction is typically effected in a polar aprotic solvent, such as N,N-dimethylformamide (DMF), dimethylsulfoxide (DMSO), N-methylpyrrolidinone, or sulfolane, at an elevated temperature ranging from about 60° C to about 250° C. Preferably, the reaction is effected in the presence of DIPEA in DMSO at between about 90° C to about 120° C.

Although Scheme 1 has been depicted as a discreet, two-step process in which intermediate (VI) is isolated and then reacted with amine (VII), it has also been found convenient to prepare and react (IV) *in situ* with amine (VII) in a single-step. In such process, an aprotic solvent, preferably DMSO, is employed. This reaction is also effected in the presence of DIPEA in DMSO at a temperature of between about 90° C to about 120° C.

### PREPARATIVE EXPERIMENTAL

Unless noted otherwise, all reagents employed were obtained commercially. Unless noted otherwise, the following experimental abbreviations have the meanings indicated:
AcOH - acetic acid
dec - decomposition
DMAP - 4-dimethylaminopyridine
EtOAc - ethyl acetate
hr - hour(s)
LAH - lithium aluminum hydride
min - minute(s)
MS - mass spectrometry
NMR - nuclear magnetic resonance
THF - tetrahydrofuran
*p*-TsOH - *p*-toluenesulfonic acid

### Preparation 1

### (2-Chloro-pyrido[2,3-d]pyrimidin-4-yl)-(3,5-dimethoxy-benzyl)-amine

To a stirred solution of 2,4 dichloro-pyrido[2,3-d]pyrimidine (1.3 g, 6.7 mmol) and 3,5-dimethoxybenzylamine (1.1 g, 6.7 mmol) in 30 mL EtOH at RT was added TEA (4 mL, 28.7 mmol). A precipitate formed that was filtered off and washed with cold EtOH followed by hexanes to give 1.8 g of the title compound (82%) as a solid. mp 185°C (dec). ¹H-NMR (DMSO-d₆) δ: 9.5 (t, 1H), 8.9 (dd, 1H), 8.7 (dd, 1H), 7.5 (m, 1H), 6.5 (d, 2H), 6.4 (t, 1H), 4.6 (d, 2H), 3.7 (s, 6H). MS (m/z, %): 331 (100).

### Preparation 2

### (2-Chloro-pyrido[2,3-d]pyrimidin-4-yl)-(3,4-dimethoxy-benzyl)-amine

This compound was prepared in a manner analogous to that described in Preparation 1 using appropriate starting materials. ¹H-NMR (DMSO-d₆) δ: 9.5 (t, 1H), 8.9 (dd, 1H), 8.7 (dd, 1H), 7.0 (s, 1H), 6.9 (d, 2H), 4.6 (d, 2H), 3.7 (s, 3H) 3.7 (s, 3H). MS (m/z, %): 331 (100).

### Preparation 3

### 2-(4-Aminomethyl-phenyl)-propan-2-ol

To a stirred solution of 4-(1-hydroxy-1-methyl-ethyl)-benzonitrile (2.0 g, 12.4 mmol) in 30 mL THF at 0°C was added dropwise 1.0 N LAH in THF (26 mL, 26.1 mmol). The mixture was allowed to warm to RT, and then refluxed for 20 min. The mixture was then cooled to 0°C and quenched with 5 mL MeOH added dropwise. The mixture was diluted with 300 mL chloroform and washed with water (1 X 80mL), dried over magnesium sulfate, and concentrated to give 1.9 g (95%) of the title compound as a solid. ¹H-NMR (CDCl₃) δ: 7.45 (d, 2H), 7.26 (d, 2H), 3.83 (s, 2H), 1.57 (s, 6H). GC-MS (m/e, %): 164 (M⁺, 15), 150 (80), 132 (75), 106 (100).

### Preparation 4

### 3-(4-Acetyl-phenyl)-propionitrile

A mixture of 4-(2-bromoethyl)acetophenone (2.0 g, 8.8 mmol) and KCN (0.6 g, 8.8 mmol) in 30 mL DMSO was heated at 75°C for 4 hr. The mixture was diluted with water and extracted with EtOAc. The organic extract was washed successively with water and brine, dried, and concentrated to give an oil. Chromatography on silica gel eluting with 40% EtOAc/hexanes gave 0.8 g of an oil. ¹H-NMR (CD₃OD) δ: 7.9 (d, 2H), 7.4 (d, 2H), 3.3 (t, 2H), 2.8 (t, 2H), 2.6 (s, 3H). GC-MS (m/e, %): 173 (M⁺, 20), 158 (100).

### Preparation 5

### 3-[4-(1-Hydroxy-1-methyl-ethyl)-phenyl]-propionitrile

To a stirred solution of 3 M methyl magnesium chloride in THF (3.3 mL, 9.8 mmol), further diluted with 10 mL THF, was added dropwise a solution of 3-(4-acetylphenyl)-propionitrile (0.7 g, 3.9 mmol) in 10 mL THF at -40°C. The reaction mixture was allowed to slowly warm to RT overnight, cooled to 0°C, then quenched with aqueous AcOH added dropwise. The reaction mixture was diluted with water and extracted with EtOAc. The organic extract was washed successively with water and brine, dried, and concentrated to give 0.8 g of an oil. ¹H-NMR (CD₃OD) δ: 7.4 (d, 2H), 7.2 (d, 2H), 2.9 (t, 2H), 2.7 (t, 2H), 1.5 (s, 6H). GC-MS (m/e, %): 189 (M⁺, 5), 174 (100).

### Preparation 6

### 2-[4-(3-Amino-propyl)-phenyl]-propan-2-ol

To a stirred solution of 1 M LAH in THF, further diluted with 20 mL THF, at 0°C was added dropwise a solution of 3-[4-(1-hydroxy-1-methyl-ethyl)-phenyl]propionitrile (0.8 g, 4.0 mmol) in 10 mL THF. The reaction was allowed to slowly warm to RT then refluxed for 4 hr. The reaction mixture was then cooled to 0°C and quenched with MeOH added slowly dropwise. The mixture was diluted with chloroform and washed with water. The organic extract was filtered through diatomaceous earth, the filtrate concentrated then diluted with ethyl acetate, dried, and concentrated to give 0.5 g of an oil. ¹H-NMR (CD₃OD) δ: 7.4 (d, 2H), 7.1 (d, 2H), 2.6 (m, 4H), 1.7 (m, 2H), 1.5 (s, 6H). MS (m/e, %): 194 (M⁺+1, 100), 176 (90).

### Preparation 7

### 3-[4-(2-Methyl-[1,3]dioxolan-2-yl)-phenyl]-propionitrile

A mixture of 3-(4-acetyl-phenyl)propionitrile (2.2 g, 13 mmol), ethylene glycol (2.8 mL, 51 mmol), and a catalytic amount of p-TsOH (~200 mg) in 100 mL toluene was refluxed over a Dean-Stark trap for 18 hr. The mixture was diluted with EtOAc and washed successively with 5% sodium bicarbonate solution, water and brine, dried (MgSO₄), and concentrated to give an oil. Chromatography on silica gel eluting with EtOAc/hexane solution gave 2.5 g of an oil. ¹H-NMR (CDCl₃) δ: 7.4 (d, 2H), 7.2 (d, 2H), 4.0 (m, 2H), 3.8 (m, 2H), 2.9 (t, 2H), 2.6 (t, 2H), 1.6 (s, 3H). MS (m/e, %): 216 (M⁺-1, 1), 202 (100).

### Preparation 8

### 3-[4-(2-Methyl[1,3]dioxolan-2-yl)-phenyl]-propylamine

To a stirred solution of 3-[4-(2-methyl-[1,3]dioxolan-2-yl)-phenyl]-propionitrile (2.4 g, 11 mmol) in 30 mL THF was added dropwise a solution of 1 M LAH in THF. The mixture was allowed to warm to RT then refluxed for 1 hr. The mixture was cooled to 0°C then quenched with MeOH added dropwise. The mixture was diluted with chloroform and washed with water. The resulting suspension was filtered through diatomaceous earth and the filtrate layers separated. The organic extract was dried over MgSO₄ and concentrated to give 2.4 g of an oil. ¹H-NMR (CDCl₃) δ: 7.4 (m, 2H), 7.1 (d, 2H), 4.0 (m, 2H), 3.8 (m, 2H), 2.7 (m, 2H), 2.6 (m, 2H), 1.7 (m, 2H), 1.6 (s, 3H). MS (m/e, %): 221 (M⁺, 10), 206 (60), 189 (100).

### Preparation 9

### 2,2,2-Trifluoro-1-(4-iodo-phenyl)-ethanol

To a stirred solution of 4-iodobenzaldehyde (2.0 g, 8.6 mmol) in 20 mL THF at RT was added 0.5M solution of trimethyl(trifluoromethyl)silane in THF (19 mL, 9.5 mmol) followed by tetrabutylammonium fluoride (112 mg, 0.4 mmol). The mixture was stirred at RT overnight, poured into 0.1 N hydrochloric acid, and extracted with EtOAc. The organic extract was in turn washed with water and brine, dried (MgSO₄), and concentrated to give 2.6 g of an oil. ¹H-NMR (CDCl₃) δ: 7.7 (d, 2H), 7.2 (d, 2H), 5.0 (m, 1H), 2.7 (d, 1H). MS (m/e, %): 302 (M⁺, 100), 233 (100).

### Preparation 10

### 2-{3-[4-(2,2,2-Trifluoro-1-hydroxy-ethyl)-phenyl]-prop-2-ynyl}-isoindole-1,3-dione

To a stirred suspension of 2,2,2-trifluoro-1-(4-iodo-phenyl)-ethanol (2.6 g, 8.5 mmol.), N-propargylphthalimide (1.6 g, 8.5 mmol.), dichlorobis(triphenylphosphine)palladium (298 mg, 0.43 mmol.) and copper (I) iodide (82 mg, 0.43 mmol) in 20 mL THF at RT was added 5 ml TEA. The mixture was deaerated briefly under a stream of nitrogen and then refluxed for 6 hr. The mixture was diluted with chloroform, washed with water, dried over MgS0₄, and concentrated to give a solid. The solid was triturated with EtOAc to afford 2.2 g of a solid. ¹H-NMR (CDCl₃) δ: 7.9 (m, 2H), 7.7 (m, 2H), 7.5 (d, 2H), 7.4 (d, 2H), 5.0 (m, 1H), 4.7 (s, 2H), MS (m/e, %): 359 (M⁺, 100).

### Preparation 11

### 2-{3-[4-(2,2,2-trilfuoro-1-hydroxy-ethyl)-phenyl]-propyl}-isoindole-1,3,dione

A mixture of 2-{3-[4-(2,2,2-trifluoro-1-hydroxy-ethyl)-phenyl]-prop-2-ynyl}-isoindole-1,3-dione (2.2 g, 6.1 mmol) and 10% Pd/C (220 mg) in 150 mL EtOH and 150 mL THF was shaken under 50 psi hydrogen at RT in a Parr apparatus for 2 hr. An additional 220 mg of 10% Pd/C was added and the mixture shaken under 50 psi hydrogen at RT for an additional 2 hr. An additional 220 mg of 10% Pd/C was then added and the mixture shaken under 50 psi hydrogen at RT overnight. The mixture was filtered through diatomaceous earth and the filtrate concentrated to give an oil. Chromatography on silica gel eluting with EtOAc/hexanes afforded 1.8 g of an oil. ¹H-NMR (CDCl₃) δ: 7.8 (m, 2H), 7.7 (m, 2H), 7.3 (d, 2H), 7.2 (d, 2H), 4.9 (m, 1H), 3.7 (t, 2H), 2.7 (t, 2H), 2.0 (m, 2H). MS (m/e, %): 363 (M⁺, 15), 345 (35), 325 (50), 161 (100).

### Preparation 12

### 1-[4-(3-Amino-propyl)-phenyl]-2,2,2-trifluoro-ethanol

To a stirred suspension of 2-{3-[4-(2,2,2-trifluoro-1-hydroxy-ethyl)-phenyl]-propyl}-isoindole-1,3-dione (1.8 g, 5.0 mmol.) in 50 mL MeOH at RT was added hydrazine hydrate (0.46 ml, 15.0 mmol). The mixture was stirred at RT for 18 hr. The reaction mixture was filtered and the filtrate concentrated to give an oil. The oil was triturated with chloroform, filtered, and the filtrate concentrated to give 1.0 g of an oil. ¹H-NMR (CDCl₃) δ: 7.3 (d, 2H), 7.2 (d, 2H), 5.0 (m, 1H), 2.6 (m, 2H), 2.5 (m, 2H), 1.6 (m, 2H). MS (m/e, %): 363 (M⁺, 10), 216 (100).

### Preparation 13

### Trifluoro-methanesulfonic acid benzo[1,2,5]oxadiazol-5-y ester

To a stirred solution of 5-hydroxybenzofurazan (1.8 g, 13.0 mmol), TEA (2.4 mL, 33.0 mmol) and DMAP (79 mg, 7.0 mmol) in 40 mL dichloromethane at -78°C was added dropwise a solution of trifluoromethanesulfonic anhydride (2.8 mL, 17.0 mmol) in 10 mL dichloromethane. The mixture was allowed to slowly warm to RT over 3 hr, diluted with dichloromethane, and washed with water. The organic extract was dried and concentrated to give an oil. Chromatography on silica gel eluting with dichloromethane afforded 3.1 g of an oil. ¹H-NMR (CDCl₃) δ: 8.0 (dd, 1H), 7.8 (dd, 1H), 7.3 (dd, 1H). MS (m/e, %): 268 (M⁺, 60), 146 (60), 69(100).

### Preparation 14

### 2-(Benzo[1,2,5]oxadiazol-5-yl-prop-2-ynyl)-isoindole-1,3-dione

This compound was prepared in a manner analogous to that in Preparation 10 using appropriate starting materials. ¹H-NMR (CDCl₃) δ: 8.2 (s, 1H), 8.0 (dd, 1H), 7.9 (m, 2H), 7.8 (m, 2H), 7.5 (dd, 2H), 4.7 (s, 2H). MS (m/e, %): 303 (M⁺, 100).

### Preparation 15

### 2-(3-Benzo[1,2,5]oxadiazol-5-yl-propyl)-isoindole-1,3,dione

This compound was prepared in a manner analogous to that in Preparation 11 using appropriate starting materials. ¹H-NMR (CDCl₃) δ: 7.8 (m, 2H), 7.7 (m, 3H), 7.6 (s, 1H), 7.2 (m, 1H), 3.8 (t, 3H), 2.8 (t, 2H), 2.1 (m, 2H). MS (m/e, %): 307 (M⁺, 40), 290(30), 272(20), 160(100).

### Preparation 16

### 3-Benzo[1,2,5]oxadiazol-5-yl-propylamine

This compound was prepared in a manner analogous to that in Preparation 12 using appropriate starting materials. ¹H-NMR (DMSO-d₆):δ 7.9 (m, 2H), 7.7 (m, 1H), 7.5 (m, 1H), 2.7 (m, 2H), 2.5 (m, 2H), 1.7 (m, 2H). MS (m/e, %): 177 (M⁺, 40), 160(100),

### Preparation 17

### Trifluoro-methanesulfonic acid benzothiazol-6-yl ester

This compound was prepared in manner analogous to that in Preparation 13 using appropriate starting materials. ¹H-NMR (CDCl₃) δ: 9.1 (s, 1H), 8.2 (d, 1H), 7.9 (d, 1H), 7.4 (dd, 1H). MS (m/e, %): 283 (M⁺, 80), 150 (100).

### Preparation 18

### 2-(3-Benzothiazol-6-yl-prop-2-ynyl)-isoindole-1,3-dione

This compound was prepared in a manner analogous to that in Preparation 10 substituting trifluoro-methanesulfonic acid benzothiazol-6-yl ester (Preparation 17) for 2,2,2-trifluoro-1-(4-iodo-phenyl)-ethanol. ¹H-NMR (d₆-DMSO) δ: 9.4 (s, 1H), 8.3 (d, 1H), 8.0 (d, 2H), 7.9 (m, 1H), 7.8 (m, 2H), 7.5 (dd, 1H), 4.6 (s, 2H). MS (m/e, %): 318 (M⁺, 100).

### Preparation 19

### 2-(3-Benzothiazol-6-yl-propyl)-isoindole-1,3-dione

This compound was prepared in a manner analogous to that in Preparation 11 substituting 2-(3-benzothiazol-6-yl-prop-2-ynyl)isoindole-1,3-dione (Preparation 19) for 2-{3-[4-(2,2,2-trifluoro-1-hydroxy-ethyl)-phenyl]-prop-2-ynyl}-isoindole-1,3-dione (Preparation 10). ¹H-NMR (CDCl₃) δ: 8.9 (s, 1H), 8.0 (d, 1H), 7.8 (m, 2H), 7.7 (m, 2H), 7.3 (dd, 1H), 3.8 (t, 2H), 2.8 (t, 2H), 2.1 (m, 2H). MS (m/e, %): 322 (M⁺, 80), 174 (40), 162 (100).

### Preparation 20

### 3-Benzothiazol-6-yl-propylamine

This compound was prepared in a manner analogous to that in Preparation 12 substituting 2-(3-benzothiazol-6-yl-propyl)-isoindole-1,3-dione (Preparation 20) for 2-{3-[4-(2,2,2-trifluoro-1-hydroxy-ethyl)-phenyl]-propyl}-isoindole-1,3-dione (Preparation 11). MS (m/e, %): 192 (M⁺, 20), 175 (100).

### Preparation 21

### 2-(3-lodo-phenyl)-2-methyl-[1,3]dioxolane

A mixture of 3-iodoacetophenone (3.0 g, 12 mmol), ethylene glycol (2.7 mL, 48 mmol), and p-toluenesulfonic acid (30 mg) in 50 mL toluene was refluxed under a Dean-Stark trap for 18 hr. The mixture was diluted with EtOAc and washed successively with 5% sodium bicarbonate solution, water, and brine, dried over MgSO₄, and concentrated to give an oil. Chromatography on silica gel eluting with EtOAc/hexanes gave 3.1 g of an oil. ¹H-NMR (CDCl₃) δ: 7.8 (m, 1H), 7.6 (dd, 1H), 7.4 (dd, 1H), 7.0 (m, 1H), 4.0 (m, 2H), 3.7 (m, 2H) 1.6 (s, 3H). MS (m/e, %): 290 (M⁺, 20), 275 (100).

### Preparation 22

### 2-{3-[3-(2-Methyl-[1,3]dioxolan-2-yl)-phenyl]-prop-2-ynyl}-isoindole-1,3-dione

This compound was prepared in a manner analogous to that in Preparation 10 substituting 2-(3-iodo-phenyl)-2-methyl-[1,3]dioxolane (Preparation 21) for 2,2,2-trifluoro-1-(4-iodo-phenyl)-ethanol. ¹H-NMR (CDCl₃) δ: 7.9 (m, 2H), 7.7 (m, 2H), 7.5 (s, 1H), 7.4 (m, 1H), 7.3 (m, 1H), 7.2 (m, 1H), 4.7 (s, 2H), 4.0 (m, 2H), 3.7 (m, 2H), 1.6 (s, 6H). MS (m/e, %): 347 (M⁺, 15), 332 (100).

### Preparation 23

### 2-{3-[3-(2-Methyl-[1,3]dioxolan-2-yl)-phenyl]-propyl}-isoindole-1,3-dione

This compound was prepared in a manner analogous to that in Preparation 11 substituting 2-{3-[3-(2-methyl-[1,3]dioxolan-2-yl)-phenyl]-prop-2-ynyl}-isoindole-1,3-dione (Preparation 22) for 2-{3-[4-(2,2,2-trifluoro-1-hydroxy-ethyl)-phenyl]-prop-2-ynyl}-isoindole-1,3-dione (Preparation 10). ¹H-NMR (CDCl₃) δ: 7.8 (m, 2H), 7.7 (m, 2H), 7.2 (m, 4H), 7.1 (d, 1H), 4.0 (m, 2H), 3.7 (m, 4H), 2.6 (t, 2H), 2.0 (m, 2H), 1.6 (s, 3H). MS (m/e, %): 351 (M⁺, 5), 336 (100).

### Preparation 24

### 3-[3-(2-Methyl-[1,3]dioxolan-2-yl)-phenyl]-propylamine

This compound was prepared in a manner analogous to that in Preparation 12 substituting 2-{3-[3-(2-methyl-[1,3]dioxolan-2-yl)-phenyl]-propyl}-isoindole-1,3-dione (Preparation 23) for 2-{3-[4-(2,2,2-trifluoro-1-hydroxy-ethyl)-phenyl]-propyl}-isoindole-1,3-dione. ¹H-NMR (CDCl₃) δ: 7.3 (m, 3H), 7.1 (dd, 1H), 4.0 (m, 2H), 3.8 (m, 2H), 2.7 (t, 2H), 2.6 (t, 2H), 1.8 (m, 2H), 1.6 (s, 3H). MS (m/e, %): 221 (M⁺, 20), 206 (60), 189 (100).

### Preparation 25

### 2-(3-Iodo-phenyl)-propan-2-ol

To a stirred solution of methyl magnesium chloride (65 mmol) in 100 mL THF at 0°C was added dropwise 3-iodoacetophenone (4.0 g, 16.3 mmol). The reaction mixture was allowed to warm to RT then cooled to 0°C and an additional equivalent of methyl magnesium chloride was added. The mixture was allowed to warm to RT and stirred for 5 hr. The reaction mixture was quenched with MeOH, diluted with water, acidified with glacial AcOH, and extracted with dichloromethane. The organic extract was washed with 5% sodium bicarbonate solution then concentrated. Chromatography on silica gel eluting with dichloromethane gave an oil which solidified upon standing. MS (m/e, %): 262 (M⁺, 80), 247 (100).

### Preparation 26

### 2-{3-[3-(1-Hydroxy-1-methyl-ethyl)-phenyl]-prop-2-ynyl}-isoindole-1,3-dione

This compound was prepared in a manner analogous to that in Preparation 10 substituting 2-(3-iodo-phenyl)-propan-2-ol (Preparation 25) for 2,2,2-trifluoro-1-(4-iodo-phenyl)-ethanol. MS (m/e, %): 319 (M⁺, 90), 301 (80), 160 (100).

### Preparation 27

### 2-{3-[3-(1-Hydroxy-methyl-ethyl)-phenyl]-propyl}-isoindole-1,3-dione

This compound was prepared in a manner analogous to that in Preparation 11 substituting 2-{3-[3-(1-hydroxy-1-methyl-ethyl)-phenyl]-prop-2-ynyl}-isoindole-1,3-dione (Preparation 26) for 2-{3-[4-(2,2,2-trifluoro-1-hydroxy-ethyl)-phenyl]-prop-2-ynyl}-isoindole-1,3-dione (Preparation 10). MS (m/e, %): 305 (M⁺-H₂O, 80), 145 (100).

### Preparation 28

### 2-[3-(3-Amino-propyl)-phenyl]-propan-2-ol

This compound was prepared in a manner analogous to that in Preparation 12 substituting 2-{3-[3-(1-hydroxy-1-methyl-ethyl)-phenyl]-propyl}-isoindole-1,3-dione (Preparation 27) for 2-{3-[4-(2,2,2-trifluoro-1-hydroxy-ethyl)-phenyl]-propyl}-isoindole-1,3-dione. MS (m/e, %): 193 (M⁺, 30), 162 (60), 145 (100).

### Preparation 29

### 4-[3-(1,3-Dioxo-1,3-dihydro-isoindol-2-yl)-prop-1-ynyl]-benzonitrile

This compound was prepared in a manner analogous to that in Preparation 10 using appropriate starting materials. ¹H-NMR (CDCl₃) δ: 7.8 (m, 6H), 7.6 (d, 2H), 4.6 (s, 2H). MS (m/e, %): 286 (M⁺, 100).

### Preparation 30

### 4-[3-(1,3-Dioxo-1,3-dihydro-isoindol-2-yl)-propyl]-benzonitrile

This compound was prepared in a manner analogous to that in Preparation 11 substituting 4-[3-(1,3-dioxo-1,3-dihydro-isoindol-2-yl)-prop-1-ynyl]-benzonitrile (Preparation 29) for 2-{3-[4-(2,2,2-trifluoro-1-hydroxy-ethyl)-phenyl]-prop-2-ynyl}-isoindole-1,3-dione (Preparation 10). ¹H-NMR (CDCl₃) δ: 7.8 (dd, 2H), 7.7 (dd, 2H), 7.5 (d, 2H), 7.3 (d, 2H), 3.7 (t, 2H), 2.7 (t, 2H), 2.0 (m, 2H). MS (m/e, %): 290 (M⁺, 60), 161 (100).

### Preparation 31

### 4-(3-Amino-propyl)-benzonitrile

This compound was prepared in a manner analogous to that in Preparation 12 substituting 4-[3-(1,3-dioxo-1,3-dihydro-isoindol-2-yl)-propyl]-benzonitrile (Preparation 30) for 2-{3-[4-(2,2,2-trifluoro-1-hydroxy-ethyl)phenyl]-propyl}-isoindole-1,3-dione. ¹H-NMR (CDCl₃) δ: 7.5 (d, 2H), 7.3 (d, 2H), 2.7 (m, 4H), 1.8 (m, 2H). MS (m/e, %): 160 (M⁺, 20), 143 (100).

### Preparation 32

### 2-Chloro-3,4-dimethoxy-benzaldehyde oxime

A mixture of 2-chloro-3,4-dimethoxybenzaldehyde (1.5 g, 7.5 mmol), hydroxylamine hydrochloride (650 mg, 9.4 mmol), and sodium acetate (1.5 g, 18.8 mmol) in 30 ml MeOH and 15 ml water was heated at 65°C for 18 hr. The mixture was diluted with water and extracted with EtOAc. The organic extract was washed successively with water and brine, dried over MgSO₄, and concentrated to give 1.7 g of a solid. ¹H-NMR (CDCl₃) δ: 8.5 (s, 1H) 7.6 (d, 1H), 6.8 (d, 1H), 3.9 (s, 3H), 3.8 (s, 3H). MS (m/e, %): 215 (M⁺, 40), 199 (100).

### Preparation 33

### 2-Chloro-3,4-dimethoxy-benzylamine

To a stirred solution of the product of Preparation 32 (1.7 g, 7.8 mmol) in 40 mL THF at 0°C was was added slowly dropwise a 1M solution of LAH in THF (17 mL, 17 mmol). The mixture was allowed to slowly warm to RT then refluxed for 2 hr. The mixture was cool to 0°C and the reaction quenched with MeOH added slowly dropwise. The mixture was diluted with water and extracted with chloroform. The resulting emulsion was filtered through diatomaceous earth and the filtrate layers separated. The organic extract was washed with water, dried (MgSO₄) and concentrated to give 1.1 g of an oil. MS (m/e, %): 202 (M⁺, 100).

### Preparation 34

### 3-Ethoxy-4-methoxy-benzaldehyde oxime

This compound was prepared in a manner analogous to that in Preparation 32 using appropriate starting materials. ¹H-NMR (CDCl₃) δ: 8.1 (s, 1H) 7.2 (d, 1H), 7.0 (m, 1H), 6.8 (d, 1H), 4.1 (q, 2H), 3.9 (s, 3H), 1.5 (t, 3H). MS (m/e, %): 195 (M⁺, 100).

### Preparation 35

### 3-Ethoxy-4-methoxy-benzylamine

This compound was prepared in a manner analogous to that in Preparation 33 using appropriate starting materials. ¹H-NMR (CDCl₃) δ: 6.9 (s, 1H) 6.8 (s, 2H), 4.1 (q, 2H), 3.8 (s, 2H), 1.5 (t, 3H). MS (m/e, %): 181 (M⁺, 100).

### Preparation 36

### 2-(3-Pyridin-4-yl-propyl)-isoindole-1,3-dione

To a stirred solution of 4-pyridinepropanol (2.0 g, 14.5 mmol), phthalimide (2.1 g, 14.5 mmol), and triphenylphosphine (4.9 g, 15.2 mmol) in 50 mL THF at 0°C was added dropwise diethyl azodicarboxylate (2.5 mL, 16.0 mmol). The mixture was allowed to slowly warm to RT then stirred overnight. The mixture was diluted with 0.1 N hydrochloric acid and washed with diethyl ether. The aqueous extract was basified with 6N sodium hydroxide and extracted with EtOAc. The organic extract was washed with 1 N sodium hydroxide and water, dried over MgSO₄, and concentrated to give 1.8 g of a solid. ¹H-NMR (CDCl₃) δ: 8.5 (s, 2H), 7.8 (m, 2H), 7.7 (m, 2H), 7.2 (m, 2H), 3.7 (t, 2H), 2.7 (t, 2H), 2.0 (m, 2H).

### Preparation 37

### 3-Pyridin-4-yl-propylamine

This compound was prepared in a manner analogous to that in Preparation 12 using the title compound of Preparation 36.¹H-NMR (CDCl₃) δ: 8.4 (m, 2H), 7.1 (m, 2H), 2.7 (t, 2H), 2.6 (t, 2H), 1.8 (m, 2H). MS (m/e, %): 136 (M⁺, 30), 119 (35), 107 (100).

The compounds of formula (I) were prepared as described in the following Examples.

### Example 1

### N⁴-(3,5-Dimethoxy-benzyl)-N²-(2-pyridin-2-yl-ethyl)-pyrido[2,3-d]pyrimidine-2,4-diamine

A mixture of the title compound of Preparation 1 (174 mg, 0.5 mmol), 2-(2-aminoethyl)-pyridine (386 mg, 3.2 mmol), and DIPEA (0.2 mL, 1.1 mmol) in 1 mL of DMSO was heated at 90°C for 18 hr. The mixture was poured into water and extracted with methylene chloride. The organic extract was washed with brine, dried over MgSO₄, and concentrated. The resulting residue was triturated with EtOAc to give 83 mg (40%) of the title compound. m.p. 157-8°C. ¹H-NMR (DMSO-d₆):δ 8.6 (dd, 1H), 8.5 (d, 1H), 8.4 (dd, 1H), 7.7 (m, 1H), 7.2 (m, 1H), 7.0 (m, 2H), 6.5 (m, 2H), 6.3 (m, 1H), 4.6 (m, 2H), 3.7 (s, 6H), 3.6 (m, 2H), 3.0 (m, 2H). MS (m/z, %): 417 (100). Anal. Calcd. for C₂₃H₂₄N₆O₂: C, 66.3; H, 5.8; N, 20.2.. Found: C, 65.3; H, 6.3; N, 18.3.

The compounds of Examples 2 to 5 were prepared in a manner analogous to that described in Example 1 using appropriate starting materials.

### Example 2

### N⁴-(3,5-Dimethoxy-benzyl)-N²-(2-pyridin-3-yl-ethyl)-pyrido[2,3-d]pyrimidine-2,4-diamine

m.p. 185-6° C. Anal. Calcd. for C₂₃H₂₄N₆O₂: C, 66.3; H, 5.8; N, 20.2. Found: C, 64.5; H, 5.6; N, 19.6.

### Example 3

### N⁴-(3,5-Dimethoxy-benzyl)-N²-(2-pyridin-4-yl-ethyl)-pyrido[2,3-d]pyrimidine-2,4-diamine

m.p. 234-5°C. Anal. Calcd. for C₂₃H₂₄N₆O₂: C, 66.3; H, 5.8; N, 20.2. Found: C, 66.0; H, 5.6; N, 20.0.

### Example 4

### N⁴-(3,5-Dimethoxy-benzyl)-N²-2-pyridin-3-ylmethyl-pyrido[2,3-d]pyrimidine-2,4-diamine

m.p. 222-3°C. Anal. Calcd. for C₂₂H₂₂N₆O₂: C, 65.7; H, 5.5; N, 20.9. Found: C, 65.5; H, 5.5; N, 20.8.

### Example 5

### N²-N⁴-Bis-(3,5-dimethoxy-benzyl)-pyrido[2,3-d]pyrimidine-2,4-diamine

m.p. 159-160°C. Anal. Calcd. for C₂₅H₂₇N₅O₄: C, 65.1; H, 5.9; N, 15.2. Found: C, 65.3; H, 5.9; N, 15.1.

### Example 6

### N⁴-(3,5-Dimethoxy-benzyl)-N²-[2-(4-methoxy-phenyl)-ethyl]-pyrido[2,3-d]pyrimidine-2,4-diamine

A mixture of the title compound of Preparation 1 (100 mg, 0.5 mmol), *p-*methoxyphenethylamine (89 µL, 0.61 mmol), and DIPEA (105 µL, 0.61 mmol) in 0.8 mL of DMSO was heated at 90°C for 18 hr. The compound was isolated and purified from the crude reaction mixture by directly injection on a reversed-phase preparative HPLC (Shimadzu Corp.; Kyoto, Japan) using a step gradient of acetonitrile/water containing 0.1% ammonium hydroxide as an elutant. Fractions containing the desired product were combined, concentrated, and the residue recrystallized from IPA to give 80 mg (60%) of the title compound. m.p. 88-9°C. Anal. Calcd. for C₂₅H₂₇N₅O₃: C, 67.4; H, 6.1; N, 15.7. Found: C, 67.0; H, 6.3; N, 15.2.

The compounds of Examples 7 to 31 were prepared in a manner analogous to that described in Example 6 using appropriate starting materials. Specific exceptions to the reaction and/or purification conditions employed are noted.

### Example 7

### N⁴-(3,5-Dimethoxy-benzyl)-N²-(3-phenyl-propyl)-pyrido[2,3-d]pyrimidine-2,4-diamine

¹H-NMR (DMSO-d₆): 8.6 (dd, 1H), 8.4 (d, 1H), 7.2 (m, br, 3H), 7.1 (m, br, 2H), 7.0 (m, br, 1H), 6.5 (s, br, 2H), 6.3 (s, br, 1H), 4.6 (m, br, 2H), 3.7 (s, br, 6H), 2.6 (m, br, 2H), 1.8 (m, br, 2H), 1.7 (m, br, 2H). MS (m/z, %): 417 (100). MS (m/z, %): 430 (100).

### Example 8

### N²-[2-(4-Chloro-phenyl)-ethyl]-N⁴-(3,5-dimethoxy-benzyl)-pyrido[2,3-d]pyrimidine-2,4-diamine

m.p. 85-90°C. Anal. Calcd. for C₂₄H₂₄N₅O₂Cl: C, 64.1; H, 5.4; N, 15.6. Found: C, 63.6; H, 5.7; N, 15.3.

### Example 9

### N²-Benzyl-N⁴-(3,5-dimethoxy-benzyl)-pyrido[2.3-d]pyrimidine-2,4-diamine

m.p. 84-85°C. Anal. Calcd. for C₂₃H₂₃N₅O₂: C, 68.8; H, 5.8; N, 17.4. Found: C, 68.5; H, 6.0; N, 17.4

### Example 10

### N⁴-(3,5-Dimethoxy-benzyl)-N²-(2-thiophen-2-yl-ethyl)-pyrido[2,3-d]pyrimidine-2,4-diamine

m.p. 120-1°C. Anal. Calcd. for C₂₂H₂₃N₅O₂S: C, 62.7; H, 5.5; N, 16.6. Found: C, 62.2; H, 6.0; N, 15.6.

### Example 11

### 2-(4-{[4-(3,5-Dimethoxy-benzylamino)-pyrido[2,3-d]pyrimidin-2-ylamino]-methyl}-phenyl)-propan-2-ol

m.p. 105-9°C. Anal. Calcd. for C₂₆H₂₉N₅O₃: C, 68.0; H, 6.4; N, 15.2. Found: C, 67.7; H, 6.6; N, 14.4.

### Example 12

### N⁴-(3,5-Dimethoxy-benzyl)-N²-2-phenethyl-pyrido[2,3-d]pyrimidine-2,4-diamine

m.p. 112-4°C. Anal. Calcd. for C₂₄H₂₅ N₅O₂: C, 69.4; H, 6.1; N, 16.9. Found: C, 68.5; H, 6.9; N, 15.3.

### Example 13

### N⁴-(3,5-Dimethoxy-benzyl)-N²-[2-(3,5-dimethoxy-phenyl)-ethyl]-pyrido[2,3-d]pyrimidine-2,4-diamine

m.p. 95-100°C. Anal. Calcd. for C₂₆H₂₉N₅O₄: C, 65.7; H, 6.2; N, 14.7. Found: C, 65.5; H, 6.6; N, 14.5.

### Example 14

### N⁴-(3,4-Dimethoxy-benzyl)-N²-[2-(3-fluoro-phenyl)-ethyl]-pyrido[2,3-d]pyrimidine-2,4-diamine

m.p. 227-8°C. Anal. Calcd. for C₂₄H₂₄N₅O₂F: C, 66.5; H, 5.6; N, 16.2. Found: C, 66.6; H, 5.6; N, 16.2.

### Example 15

### N⁴-(3,4-Dimethoxy-benzyl)-N²-[2-(2-fluoro-phenyl)-ethyl]-pyrido[2,3-d]pyrimidine-2,4-diamine

m.p. 220-2°C. Anal. Calcd. for C₂₄H₂₄N₅O₂F: C, 66.5; H, 5.6; N, 16.2. Found: C, 66.4; H, 5.5; N, 16.1.

### Example 16

### N⁴-(3,4-Dimethoxy-benzyl)-N²-[2-(4-fluoro-phenyl)-ethyl]-pyrido[2,3-d]pyrimidine-2,4-diamine

m.p. 225-6°C. Anal. Calcd. for C₂₄H₂₄N₅O₂F: C, 66.5; H, 5.6; N, 16.2. Found: C, 66.4; H, 5.6; N, 16.2.

### Example 17

### N⁴-(3,4-Dimethoxy-benzyl)-N²-phenethyl-pyrido[2,3-d]pyrimidine-2,4-diamine

m.p. 217-8°C. Anal. Calcd. for C₂₄H₂₅N₅O₂: C, 69.4; H, 6.1; N, 16.9. Found: C, 69.4; H, 6.0; N, 16.8.

### Example 18

### N⁴-(3,4-Dimethoxy-benzyl)-N²-(4-phenyl-butyl)-pyrido[2,3-d]pyrimidine-2,4-diamine

m.p. 194-5°C. Anal. Calcd. for C₂₆H₂₉N₅O₂: C, 70.4; H, 6.6; N, 15.8. Found: C, 70.5; H, 6.6; N, 15.9.

### Example 19

### N⁴-(3,4-Dimethoxy-benzyl)-N²-(2-phenoxy-ethyl)-pyrido[2,3-d]pyrimidine-2,4-diamine

m.p. 227-8°C. Anal. Calcd. for C₂₄H₂₅N₅O₃: C, 66.8; H, 5.8; N, 16.2. Found: C, 66.7; H, 5.7; N, 16.2.

### Example 20

### N⁴-(3,4-Dimethoxy-benzyl)-N²-(2-trifluoromethyl-benzyl)-pyrido[2,3-d]pyrimidine-2,4-diamine

m.p. 227-8°C. Anal. Calcd. for C₂₄H₂₂N₅O₂F₃: C, 61.4; H, 4.7; N, 14.9. Found: C, 60.6; H, 4.9; N, 14.8.

### Example 21

### 2-(4-{3-[4-(3,4-Dimethoxy-benzylamino)-pyrido[2,3-d]pyrimidin-2-ylamino]-propyl}-phenyl)-propan-2-ol

Reversed-phase preparative HPLC fractions containing crude desired product were concentrated and the residue was recrystallized from EtOAc. m.p. 198-9°C. Anal. Calcd. for C₂₈H₃₃N₅O₃: C, 69.0; H, 6.8; N, 14.4. Found: C, 68.7; H, 6.9; N, 14.3.

### Example 22

### 2-(4-{3-[4-(3,5-Dimethoxy-benzylamino)-pyrimidin-2-ylamino-2-ylamino]-propyl}-phenyl)-propan-2-ol

(amorphous solid) m.p. 70-5°C. ¹H-NMR (DMSO-d₆): 8.6 (dd, 1H), 8.4 (d, 1H), 7.3 (m, br, 2H), 7.1 (m, br, 2H), 7.0 (m, br, 2H), 6.5 (s, br, 2H), 6.3 (s, br, 1H), 4.6 (m, br, 2H), 3.7 (s, br, 6H), 2.6 (m, br, 2H), 1.8 (m, br, 2H), 1.7 (m, br, 2H), 1.4 (s 6H). MS (m/z, %): 430 (100). MS (m/z, %): 488 (M⁺+1, 100), 470 (50). Anal. Calcd. for C₂₈H₃₃N₅O₃: C, 69.0; H, 6.8; N, 14.4. Found: C, 67.1; H, 7.0; N, 12.1.

### Example 23

### 1-(4-{[4-(3,4-Dimethoxy-benzylamino)-pyrido[2,3-d]pyrimidin-2-ylamino]-methyl}-phenyl)-2,2,2-trifluoro-ethanol

Reversed-phase preparative HPLC fractions containing crude desired product were concentrated and the residue was recrystallized from acetonitrile/water. m.p. 121-2°C. Anal. Calcd. for C₂₅H₂₄N₅O₃F₃: C, 60.1; H, 4.8; N, 14.0. Found: C, 58.0; H, 5.0; N,13.4

### Example 24

### 1-(4-{3-[4-(3 4-Dimethoxy-benzylamino)-pyrido[2,3-d]pyrimidin-2-ylamino]-propyl}-Phenyl)-ethanone

The reaction mixture containing crude product was poured into water and the resulting precipitate was filtered off. The residue was dissolved in 30 mL of MeOH and 10 mL of 1 N hydrochloric acid was added. The mixture was stirred at RT for four hr, concentrated, neutralized with 5% sodium bicarbonate solution, and extracted with dichloromethane. The organic extracts were combined and washed with brine, dried over MgSO₄, and concentrated. The crude product was purified by reversed-phase HPLC as described in Example 6. Fractions containing desired product were combined, concentrated, and the residue recrystallized from acetonitrile/water to furnish a solid. m.p. 184-5°C. Anal. Calcd. for C₂₇H₂₉N₅O₃: C, 68.8; H, 6.2; N, 14.9. Found: C, 68.7; H, 6.0; N, 14.6.

### Example 25

### 1-(4-{3-[4-(3,4-Dimethoxy-benzylamino)-pyrido[2,3-d]pyrimidin-2-ylamino]-propyl}-phenyl)-2,2,2-trifluoro-ethanol

m.p. 222-4°C. ¹H-NMR (DMSO-d₆):δ 8.6 (dd, 1H), 8.4 (dd, 1H), 7.4 (m, br, 2H), 7.3 (m, 1H), 7.0 (m, br, 2H), 6.8 (s, br, 2H), 6.7 (d, 1H), 5.0 (m, br, 1H), 4.6 (s, br, 2H), 3.7 (m, 6H), 3.3 (m, 2H), 2.6 (m, br, 2H), 1.8 (m, br, 2H). MS (m/z, %): 528 (M⁺, 100).

### Example 26

### N²-3-(Benzo[1,2,5]oxadiazol-5-yl-propyl)-N⁴-(3,4-dimethoxy-benzyl)-pyrido[2,3-d]pyrimidine-2,4-diamine

The product was isolated by chromatography on silica gel eluting with 2.5% MeOH in 2 N ammonia/dichloromethane to give a crude solid. Trituration with EtOAc afforded a solid. m.p. 191-3°C. ¹H-NMR (DMSO-d₆):δ 8.6 (dd, 1H), 8.4 (dd, 1H), 7.9 (m, br, 1H), 7.7 (m, 1H), 7.5 (m, br, 1H), 7.0 (m, br, 2H), 6.8 (m, 2H), 4.6 (m, br, 2H), 3.7 (m, 6H), 3.4 (m, 2H), 2.7 (m, br, 2H), 1.9 (m, br, 2H). MS (m/z, %): 472 (M⁺, 100).

### Example 27

### N²-3-(Benzothiazol-6-yl-propyl)-N⁴-(3,4-dimethoxy-benzyl)-pyrido[2,3-d]pyrimidine-2.4-diamine

The product was isolated by chromatography on silica gel eluting with 2.5% MeOH in 2 N ammonia/dichloromethane to give a solid. mp 191-3°C. MS (m/z, %): 487 (M⁺, 100). Anal. Calcd. for C₂₆H₂₆N₆O₂S: C, 64.2; H, 5.4; N, 17.3. Found: C, 64.5; H, 5.5; N, 16.8.

### Example 28

### N⁴-(3,4-Dimethoxy-benzyl)-N²-{3-[3-(2-methyl-[1,3]-dioxolan-2-yl)-phenyl]-propyl}-pyrido[2,3-d]pyrimidine-2,4-diamine

¹H-NMR (DMSO-d₆):δ 8.6 (d, 1H), 8.3 (d, 1H), 7.2 (m, br, 3H), 7.0 (m, br, 3H), 6.9 (m, br, 2H), 4.6 (m, br, 2H), 4.1 (m, 2H), 3.9 (m, 2H), 3.7 (m, br, 8H), 2.6 (m, br, 2H), 1.8 (m, br, 2H)1.5 (s, 3H). MS (m/z, %): 516 (M⁺+1, 100).

### Example 29

### 2-(3-{3-[4-(3,4-Dimethoxy-benzylamino)-pyrido[2,3-d]pyrimidin-2-ylamino]-propyl}-phenyl)-propan-2-ol

m.p. 204-5°C. ¹H-NMR (DMSO-d₆): δ 8.6 (d, 1H), 8.3 (d, 1H), 7.3 (m, br, 1H), 7.2 (m, br, 1H), 7.1 (m, br, 1H), 7.0 (m, br, 3H), 6.8 (s, br, 2H), 4.6 (s, br, 2H), 3.7 (m, br, 6H), 3.3 (m, br, 2H), 2.6 (m, br, 2H), 1.8 (m, br, 2H), 1.4 (s, 6H). MS (m/z, %): 488 (M⁺+1, 100).

### Example 30

### 4-{3-[4-(3,4-Dimethoxy-benzylamino)-pyrido[2,3-d]pyrimidin-2-ylamino]-propyl}-benzonitrile

m.p. 220-2°C. ¹H-NMR (DMSO-d₆):δ 8.6 (d, 1H), 8.3 (d, 1H), 7.7 (m, br, 2H), 7.4 (m, br, 2H), 7.0 (m, br, 2H), 6.8 (s, br, 2H), 4.6 (s, br, 2H), 3.7 (m, br, 6H), 3.3 (m, br, 2H), 2.7 (m, br, 2H), 1.8 (m, br, 2H). MS (m/z, %): 455 (M⁺+1, 100).

### Example 31

### N⁴-(3,5-Dimethoxy-benzyl)-N²-(3-pyridin-4-yl-propyl)-pyrido[2,3-d]pyrimidine-2,4-diamine

¹H-NMR (CD₂Cl₂): δ 9.1 (d, 1H), 8.9 (m, br, 1H), 8.4 (m, br, 3H), 7.2 (m, br, 2H), 7.1 (m, br, 1H), 7.0 (s, br, 1H), 6.9 (d, 1H), 6.6 (d, 1H), 4.8 (d, 2H), 3.7 (s, 3H), 3.7 (s, 3H), 3.6 (m, 2H), 2.7 (m, 2H), 2.0 (m, 2H). MS (m/z, %): 432 (M⁺+1, 20), 256 (15), 237 (30), 216 (100).

The compounds of Examples 32-35 were prepared according to the following generalized procedure. Specific exceptions to the reaction and/or purification conditions employed are noted.

To a stirred solution of 2,4-dichloro-pyrido[2,3-d]pyrimidine (0.75 mmol) and DIPEA (1.5 mmol) in 3 mL DMSO was added 0.75 mmol of an amine corresponding to formula (V), Scheme 1, STEP 1, at RT. The mixture was stirred at RT for one hr, then 2.25 mmol of an amine corresponding to formula (VII), Scheme 1, STEP 2, and additional DIPEA (2.25 mmol) were added, and the mixture heated at 90°C for two hr.

### Example 32

### N⁴-(3,4-Dimethoxy-benzyl)-N²-(3-phenyl-propyl)-pyrido[2,3-d]pyrimidine-2,4-diamine

The compound was isolated and purified from the crude reaction mixture by direct injection onto a reversed-phase preparative HPLC using a step gradient of acetonitrile/water containing 0.1 % ammonium hydroxide as an elutant. Fractions containing the compound were combined and concentrated to give a solid. m.p. 190-2°C. ¹H-NMR (d₆-DMSO): δ 8.6 (dd, 1H), 8.4 (dd, 1H), 7.2 (m, br, 3H), 7.1 (m, br, 2H), 7.0 (m, br, 2H), 6.8 (m, br, 2H), 4.6 (m, br, 2H), 3.7 (d, br, 6H), 3.3 (m, br, 2H), 2.6 (m, br, 2H), 1.8 (m, 2H). MS (m/e, %): 431 (M⁺+1, 50), 430 (M⁺, 100).

### Example 33

### N⁴-(3,5-Dimethoxy-benzyl)-N²-(3-phenoxy-ethyl)pyrido[2,3-d]pyrimidine-2,4-diamine

The compound was isolated and purified from the crude reaction mixture by direct injection onto a reversed-phase preparative HPLC using a step gradient of acetonitrile/water containing 0.1% ammonium hydroxide as an elutant. Fractions containing the compound were combined, concentrated, and the residue recrystallized from IPA/water. m.p. 165-7°C. ¹H-NMR (d₆-DMSO): 8.6 (dd, 1H), 8.4 (d, 1H), 7.2 (m, br, 3H), 7.1 (m, br, 2H), 7.0 (m, 1H), 6.9 (m, br, 2H), 6.8 (m, br, 1H), 6.5 (d, 2H), 6.4 (s, 1H), 4.6 (m, br, 2H), 4.0 (m, br, 2H), 3.7 (m, br, 8H). MS (m/e, %): 433 (M⁺+1, 50), 432 (M⁺, 100).

### Example 34

### N⁴-(3-Ethoxy-4-methoxy-benzyl)-N²-(3-phenyl-propyl)-pyrido[2,3-d]pyrimidine-2,4-diamine

The compound was isolated and purified from the crude reaction mixture by direct injection onto a reverse phase preparative HPLC using a step gradient of acetonitrile/water containing 0.1% ammonium hydroxide as an elutant. Fractions containing the compound were combined, concentrated, and recrystallized from acetonitrile/water. m.p. 181-2°C. Anal. Calcd. for C₂₆H₂₉N₅O₂: C, 70.4; H, 6.6; N, 15.8. Found: C, 70.7; H, 6.9; N, 15.9.

### Example 35

### 2-(4-{3-[4-(3-Ethoxy-4-methoxy-benzylamino)-pyridor[2,3-d]pyrimidin-2-ylamino]-propyl}-phenyl)-propan-2-ol

The compound was isolated and purified from the crude reaction mixture by direct injection onto a reverse phase preparative HPLC using a step gradient of acetonitrile/water containing 0.1% ammonium hydroxide as an elutant. Fractions containing the desired compound were combined, concentrated, and recrystallized from acetonitrile/water. m.p. 150-2°C. Anal. Calcd. for C₂₉H₃₅N₅O₃: C, 69.4; H, 7.0; N, 13.7. Found: C, 69.1; H, 6.9; N, 13.7.

### Example 36

### 1-(4-{3-[4-(3,4-Dimethoxy-benzylamino)-pyrido[2,3-d]pyrimidin-2-ylamino]-propyl}-phenyl)-2,2,2-trifluoro-ethanone

To a stirred stirred solution of the title compound of Example 25 (150 mg, 0.28 mmol) in 15 mL dichloromethane at RT was added 1,1,1-triacetoxy-1,1-dihydro-1,2-benziodoxol-3(1H)-one (180 mg, 0.43 mmol). The mixture was stirred at RT for six hr, then an additional portion of 1,1,1-triacetoxy-1,1-dihydro-1,2-benziodoxol-3(1H)-one (180 mg, 0.43 mmol) was added and the mixture stirred at RT overnight. The mixture was diluted with chloroform, washed with water, dried over MgSO₄, and concentrated to give an oil. Chromatography on silica gel eluting with 5% MeOH 2N in ammonia/dichloromethane afforded an oil. Trituration with EtOAc yielded 45 mg of a solid. m.p. 198-200°C. ¹H-NMR (d₆-DMSO): δ 8.6 (dd, 1H), 8.4 (dd, 1H), 7.9 (m, br, 2H), 7.5 (m, br, 2H), 7.0 (m, br, 2H), 6.8 (m, br, 2H), 4.6 (m, 2H), 3.7 (m, 6H), 3.3 (m, 2H), 2.7 (m, br, 2H), 1.9 (m, br, 2H). MS (m/e, %): 526 (M⁺, 100).

### Example 37

### 1-(3-{3-[4-(3,4-Dimethoxy-benzylamino)-pyrido[2,3-d]pyrimidin-2-ylamino]-propyl}-phenyl)-ethanone

A mixture of the title compound of Preparation 27 (419 mg, 0.81 mmol) in 8 mL of THF containing 10 mL of 6 N hydrochloric acid was stirred at RT for six hr. The reaction mixture was extracted with EtOAc, the organic extract was washed with 5% sodium bicarbonate solution, and then concentrated to give a solid. m.p. 152-5°C. ¹H-NMR (d₆-DMSO): 8.8 (d, 1H), 8.7 (d, 1H), 7.7 (m, 2H), 7.4 (m, 3H), 7.0 (s, 1H), 6.8 (m, 2H), 4.6 (d, 2H), 3.7 (s, 3H), 3.6 (s, 3H), 3.5 (m, 2H), 2.7 (m, 2H), 2.5 (s, 3H), 1.9 (m, 2H). MS (m/e, %): 471 (M⁺, 30), 470 (100).

### BIOLOGICAL METHODOLOGIES

### PDE 2 Enzyme Isolation

PDE 2 enzyme was isolated from human platelets with approximately 1.4 L of blood from multiple donors used to make the platelet pellets. Platelets were resuspended in approximately 75 mL of lysis buffer [20 mM Tris pH 7.2, 5 mM MgCl₂, 250 mM Sucrose, 1 mM DTT, 1 µL/2 mL Sigma Protease inhibitor #8340; Sigma-Aldrich; St. Louis, MO] and lysed by sonication at 4°C, with 3 rounds of 1 min bursts then spun at 4°C overnight at 100,000x g. Cleared lysates were loaded to AKTA Explorer FPLC (Amersham Biosciences; Piscataway, NJ) in a series of three chromatographic separations, with an average load volume of 25 mL. A 5 mL HiTrap Q anion exchange column (Amersham Biosciences) was used. A buffer [20 mM Tris pH 7.2, 5 mM MgCl₂, 1 µl/2 mL Sigma Protease inhibitor #8340] was mixed with a gradient of B Buffer [20 mM Tris pH 7.2, 500 mM NaCl, 5 mM MgCl₂, 1 µl/2 mL Sigma Protease inhibitor #8340] over 20 column volumes from an initial B Buffer concentration of 0% to a final concentration of 100%. cGMP-hydrolyzing peaks were noted with average resolution at low salt- 125 mM NaCl (PDE 5) and high salt- 325 mM NaCl (PDE 2). Two major cGMP activity fractions (PDE 5 and PDE 2) were isolated and pooled separately. The PDE 2-pooled fraction total was approximately 40 mL which was dispensed in cryovials of 200 µl/vial and placed in storage at -80°C.

### PDE 2 Enzyme Binding Assay

The inhibitory activity of the compounds of formula (I) on recombinant or isolated PDE 2, and other PDE's, was determined using the [³H]cAMP scintillation proximity assay (SPA) kits from Amersham International (Little Chalfont, England). The SPA assays were performed using 96 well plates. The PDE SPA yttrium silicate beads (Amersham Biosciences) bind preferentially to the linear nucleotide, GMP, compared to the cyclic nucleotide, cGMP. ³H-cGMP was added to the reaction and when the product, ³H-GMP, was in dose proximity to the beads, the scintillant within the bead was excited, which was detected using a Packard scintillation counter (Perkin-Elmer Life Sciences; Boston, MA). The enzyme concentration used was in the linear range and the Kₘ of the enzyme was determined (15 uM). The final substrate concentration was <1/3 of Kₘ (1 µM) so that IC₅₀ values would approximate the Kᵢ values. The assay was validated using literature compounds as controls before testing compounds. Then, PDE catalytic activity measurements obtained in the presence of the test compound, and those obtained in the absence of the test compound, were compared and the IC₅₀ value is determined.

The radioactive substrates and the products of the PDE reaction were determined quantitatively using a RACK-BETA 1219 liquid scintillation counter (LKB Wallac; Freiburg, FRG). The IC₅₀ values (concentrations with 50% inhibition) were determined with 1µM cAMP or cGMP using the peak fractions. The data were fitted with four parameters with the aid of the sigmoidal logistic function.

Using the previously described PDE 2 enzyme isolated from human platelets and the method for assaying test compounds for inhibition of the enzyme, an IC₅₀ of 1.7 uM was determined for EHNA. In addition, an IC₅₀ of 3 nM for 9-(1-acetyl-4-phenyl-butyl)-2-(3,4-dimethoxy-benzyl)-1,9-dihydropurin-6-one was also determined using the aforementioned method.

The compounds of formula (I) generally exhibit inhibitory activity, expressed as IC₅₀'s against PDE 2, that are <1,000 nM. Ranges of PDE 2 inhibitory activity for the compounds of formula (I) in Examples 1-37 are set forth in Table 1.

**Table 1**

| Example | PDE 2 Inhibition | Example | PDE 2 Inhibition |
|---|---|---|---|
| 1 | ++ | 22 | ++ |
| 2 | ++ | 23 | + |
| 3 | + | 24 | + |
| 4 | + | 25 | ++ |
| 5 | +++ | 26 | + |
| 6 | + | 27 | + |
| 7 | ++ | 28 | + |
| 8 | + | 29 | + |
| 9 | + | 30 | + |
| 10 | +++ | 31 | + |
| 11 | + | 32 | + |
| 12 | ++ | 33 | ++ |
| 13 | ++ | 34 | + |
| 14 | ++ | 35 | + |
| 15 | ++ | 36 | + |
| 16 | +++ | 37 | + |
| 17 | +++ | | |
| 18 | ++ | | |
| 19 | ++ | | |
| 20 | + | | |
| 21 | + | | |

| | | | |
|---|---|---|---|
| PDE 2 Inhibition: +++ (IC₅₀<50 nM), ++ (lC₅₀ 50-250 nM), + (IC₅₀ 250-1,000 nM) | | | |

The ability of PDE 2 inhibitors, including the compounds of formula (I), to treat bone fracture and/or defect, or promote bone in-growth, may be demonstrated according to the following protocols.

### Rat Transverse Femoral Fracture Model

Male Sprague-Dawley rats at 3 to 4 months of age were used. The animals were anesthetized with ketamine and xylazine at doses of 100 and 10 mg/kg, respectively. The right hindlimb of each rat was shaved and cleaned. A 1 cm incision was made just lateral to the patella and the femoral condyle was exposed. A Kirschner wire (0.045" in diameter) was introduced into the intramedullary canal through the intercondylar portion to serve as an internal stabilization. The muscle incision was closed with vicryl and the skin incision was closed with stainless steel wound clips. The mid-diaphysis of the pinned femur was fractured by means of a three-point bending device driven by a dropped weight. The rats were permitted full weight-bearing and unrestricted activity after awakening from anesthesia. The test compounds were administered on various days after surgery by percutaneous injection onto the fracture site. The animals were sacrificed after treatment and the femurs were collected for analysis. Fracture healing was evaluated by using radiography, histological, and biomechanical test (F. Bonnarens, et al., Journal of Orthopaedic Research, 2, 97-101 (1984).

### Study Protocol and Results in the Rat Transverse Femoral Fracture Model

Three month-old male rats were subjected to transverse fracture of their right femurs under general anesthesia. A single dose (5 mg) of *N*⁴-(3,4-dimethoxy-benzyl)-*N*²-(3-phenyl-propyl)-pyrido[2,3-d]pyrimidine-2,4-diamine was percutaneously injected into the fracture sites at the completion of fracture generation. Three weeks after the injections, the rats were sacrificed and the right femurs were harvested and analyzed. Force-to-failure and stiffness (indices of bone strength) were increased by 19% and 62%, respectively, in the femurs treated with *N*⁴-(3,4-dimethoxy-benzyl)-*N*²-(3-phenylpropyl)-pyrido[2,3-d]pyrimidine-2,4-diamine, compared to those of the femurs treated with placebo.

### Rat Periosteal Injection Model

Rats were anesthetized with isoflurane (2-3 min) in a conduction chamber located in a fume hood. The right hindlimb of each rat was shaved and cleaned. A 25 gauge needle attached to a syringe was pre-filled with a formulation of the test compound for local injection. The formulation was injected onto the subperiosteum of femur in a volume of 5-15 µL for 14 days. The rats were sacrificed after dosing, the femurs were collected, and then analyzed by radiography and dual-energy X-ray absorptiometry (DEXA).

### Study Protocol and Results in the Rat Periosteal Injection Model

The right femur of three-week old male Sprague-Dawley rats received a daily injection of vehicle or test compound five times per week for two weeks. On Day 15, all rats were sacrificed and the right femurs were collected for analysis. Periosteal bone induction was assessed using radiography and DEXA. Radiography showed new bone formation located on the injection site of all femurs treated with test compound. The bone mineral content (BMC) of the injected region of the femur (area between lesser trochanter and mid-shaft of the femur) was assessed by DEXA comparing rats treated with test compound with those treated only with vehicle. In this model, *N*⁴-(3,5-dimethoxy-benzyl)-*N*²-(2-pyridin-4-yl-ethyl)-pyrido[2,3-d]pyrimidine-2,4-diamine, 2-(3-{3-[4-(3,4-dimethoxy-benzylamino)-pyrido[2,3-d]pyrimidin-2-ylamino]-propyl}-phenyl)-propan-2-ol, and *N*⁴-(3,4-dimethoxy-benzyl)-*N*²-(3-phenyl-propyl)-pyrido[2,3-d]pyrimidine-2,4-diamine increased BMC by 34%, 49%, and 33% respectively. Additionally, 9-(1-acetyl-4-phenyl-butyl)-2-(3,4-dimethoxy-benzyl)-1,9-dihydropurin-6-one increased BMC by 20% in the aforementioned model.

## Claims

1. A compound of formula (I) 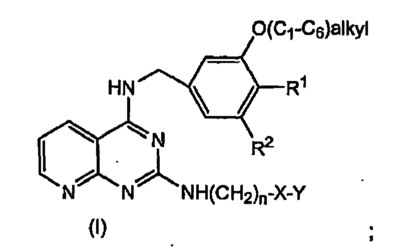 and the pharmaceutically acceptable salts of said compounds wherein:
R¹ and R² are hydrogen or methoxy, provided R¹ and R² are not both hydrogen or both methoxy;
n is 1, 2, 3, or 4;
X is a bond; O; S; C=O; -N(R)-, wherein R is hydrogen or -(C₁- C₃)alkyl; -C(OH)-; or -SO₂; and
Y is benzoxazolyl; benzothlazolyl; benzofurazanyl; benzofuranyl; benzothiadiazolyl; benzisoxazolyl; benzisothiazolyl; benzimidazolyl; pyridyl; isatinyl; oxindolyl; indazolyl; indolyl; phenyl; thienyl; or furanyl; wherein Y is optionally substituted independently with from one to three halogen; trifluoromethyl; methoxy; -C(=O)CH₃; cyano; -C(CH₃)₂OH; -CH(CH₃)OH; -CH(CF₃)OH; -C(C=O)CF₃; -SO₂NH₂; -C(=O)OCH₃; -CH₂COOH; 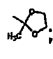 thiazolyl; or oxadiazolyl.

2. A compound of claim 1, wherein X is a bond, and Y is benzofurazanyl; thienyl; pyridyl; or phenyl, wherein phenyl is optionally substituted independently with one or two halogen; trifluoromethyl; methoxy; -C(=O)CH₃; cyano; -C(CH₃)₂OH; -CH(CH₃)OH; -CH(CF₃)OH; -C(C=O)CF₃; -SO₂NH₂; -C(=O)OCH₃; -CH₂COOH; thiazolyl; or oxadiazolyl.

3. A compound of claim 1 or 2, wherein X is a bond, n is 2 or 3, and Y is thienyl; pyridyl; or phenyl, wherein phenyl is optionally substituted independently with one or two methoxy; halogen; -C(CH₃)₂OH; CH(CF₃)OH; or -C(C=O)CF₃.

4. *N²,N*⁴-*bis*-(3,5-Dimethoxy-benzyl)-pyrido[2,3-d]pyrimidine-2,4-diamine;
*N*⁴-(3,5-dimethoxy benzyl)-*N²*-(2-pyridin-4-yl-ethyl)-pyrido[2,3-d]pyrimidine-2,4-dlamine;
*N⁴*-(3,5-dimethoxy-benzyl)-*N²*-(2-thlophen-2-yl-ethyl)-pyrido[2,3-d]pyrimidine-2,4-diamine;
*N⁴*-(3,5-dimethoxy-benzyl)-*N²*-2-phenethyl-pyrido[2,3-d]pyrimidine-2,4-diamine;
*N*⁴-(3,5-dimethoxy-benzyl)-*N²*-[2-(3,5-dimethoxy-phenyl)-ethyl]-pyrido[2,3-d]pyrimidine-2,4-diamine;
2-(3-{3-[4-(3,4-dimethoxy-benzylamino)-pyrido[2,3-d]pyrimidin-2-ylamino]-propyl}-phenyl)-propan-2-ol;
*N*⁴-(3,4-dimethoxy-benzyl)-*N*²-[2-(4-fluoro-phenyl)-ethyl]-pyrido[2,3-d]pyrimidlne-2,4-diamine;
*N*⁴-(3,4-dimethoxy-benzyl)-*N*²-phenethyl-pyrido[2,3-d]pyrimidine-2,4-diamine; or
*N*⁴-(3,4-dimethoxy-benzyl)-*N*²-(3-phenyl-propyl)-pyrido[2,3-d]pyrimidine-2,4-diamine; or a pharmaceutically acceptable salt of said compound.

5. A pharmaceutical composition comprising a compound of formula (I) of any of claims 1-4, or a pharmaceutically acceptable salt of said compound and a pharmaceutically acceptable vehicle, carrier, or diluent

6. The use of a compound or salt according to any one of claims 1 to 4 in the manufacture of a medicament for treating a PDE 2-mediated condition, disease, or symptom in a mammal.

7. The use of claim 6, wherein said condition, disease, or symptom is osteoporosis, pulmonary hypertension, female sexual arousal disorder, diminished memory or cognition, platelet aggregation, vascular angiogenesis, dementia, cancer, arrhythmia, thrombosis, bone fracture and/or defect, delayed or non-union fracture, spinal fusion, bone in-growth, cranial facial reconstruction, or hypoxia.

8. The use of claim 6, wherein said condition is bone fracture and/or defect.

9. A pharmaceutical composition comprising a PDE 2 inhibitor, according to formula (I) of claim 1 an EP₂ selective receptor agonist, and a pharmaceutically acceptable vehicle, carrier, or diluent.

10. A composition of claim 9, wherein said PDE 2 inhibitor is *N*⁴-(3,5-dimethoxybenzyl)-*N*²-(2-pyridin-4-yl-ethyl)-pyrido[2,3,d]pyrimidin-2,4-diamine; 2-(3-{3-[4-(3,4-dimethoxy-benzylamino)-pyrido[2,3-d]pyrimidin-2-ylamino]-propyl}-phenyl)-propan-2-ol; *N*⁴-(3,4-dmethoxy-benzyl)-*N*²-(3-phenyl-propyl)-pyrido[2,3-d]pyrimidine-2,4-diamine; or a pharmaceutically acceptable salt of said compound.

11. A composition of claim 9 or 10, wherein said said EP₂ selective receptor agonist is (3-(((4-*tert-*butyl-benzyl)-(pyridine-3-sulfonyl)-amino)-methyl)-phenoxy)-acetic acid, or a pharmaceutically acceptable salt of said compound.

12. The use of any of claims 6-8, which treatment further comprises administering to said mammal a therapeutically effective amount of an EP₂ selective receptor agonist; or a pharmaceutical composition comprising a combination of said compound of formula (I) of claim 1 and said EP₂ selective receptor agonist.

13. The use of claim 12, wherein said PDE 2 Inhibitor is *N*⁴-(3,5-dimethoxy-benzyl)-*N*²-(2-pyridin-4-yl-ethyl)pyrido[2,3-d]pyrimidin-2,4-diamine; 2-(3-{3-[4-(3,4-dimethoxybenzylamino)-pyrido[2,3-d]pyrimidin-2-ylamino]-propyl}-phenyl)-propan-2-ol, *N*⁴-(3,4-dimethoxy-benzyl)-*N*²-(3-phenyl-propyl)-pyrido[2,3-d]pyrimidine-2,4-diamine; or a pharmaceutically acceptable salt of said compound.

14. The use of claim 12 or 13, wherein said EP₂ selective receptor agonist is (3-(((4-*tert*-butyl-benzyl)-(pyridine-3-sulfonyl)-amino)-methyl)-phenoxy)-acetic acid, or a pharmaceutically acceptable salt of said compound.

15. The use of a PDE 2 inhibitor according to formula (I) of claim 1 or a pharmaceutically acceptable salt of said inhibitor, in the manufacture of a medicament for the treatment of a bone fracture and/or defect in a mammal.

16. A compound or salt according to claim 1 for use as a medicament.

17. A compound or salt according to claim 1 for use as a medicament in the treatment of a PDE 2-mediated condition, disease or symptom in a mammal.

## Patentansprüche

1. Verbindung der Formel (I) 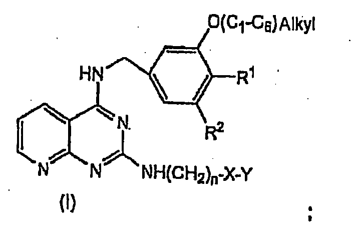 und die pharmazeutisch annehmbaren Salze der Verbindungen, worin:
R¹ und R² Wasserstoff oder Methoxy sind, mit der Maßgabe, dass R¹ und R² nicht beide Wasserstoff oder beide Methoxy sind;
n 1, 2, 3 oder 4 ist;
X eine Bindung, O, S, C=O, -N(R)- ist, wobei R Wasserstoff oder -(C₁-C₃)Alkyl, -C(OH)- oder -SO₂ ist, und
Y Benzoxazolyl, Benzothiazolyl, Benzofurazanyl, Benzofuranyl, Benzothiadiazolyl, Benzisoxazolyl, Benzisothiazolyl, Benzimidazolyl, Pyridyl, Isatinyl, Oxindolyl, Indazolyl, Indolyl, Phenyl, Thienyl oder Furanyl, wobei Y gegebenenfalls unabhängig substituiert ist mit von eins bis drei Halogen, Trifluormethyl, Methoxy, -C(=O)CH₃, Cyano, -C(CH₃)₂OH, -CH(CH₃)OH, -CH(CF₃)OH, -C(C=O)CF₃, -SO₂NH₂, -C(=O)OCH₃, -CH₂COOH, 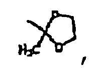 Thiazolyl oder Oxadiazolyl, ist.

2. Verbindung nach Anspruch 1, worin X eine Bindung und Y Benzofurazanyl, Thienyl, Pyridyl oder Phenyl, wobei Phenyl gegebenenfalls unabhängig substituiert ist mit einem oder zwei Halogen, Trifluormethyl, Methoxy, -C(=O)CH₃, Cyano, -C(CH₃)₂OH, -CH(CH₃)OH, -CH(CF₃)OH, -C(C=O)CF₃, -SO₂NH₂, -C(=O)OCH₃, -CH₂COOH, Thiazolyl oder Oxadiazolyl, ist.

3. Verbindung nach Anspruch 1 oder 2, worin X eine Bindung ist, n 2 oder 3 ist und Y Thienyl, Pyridyl oder Phenyl, wobei Phenyl gegebenenfalls unabhängig substituiert ist mit einem oder zwei Methoxy, Halogen, -C(CH₃)₂OH, CH (CF₃) OH oder -C(C=O)CF₃, ist.

4. N²,N⁴-Bis-(3,5-dimethoxybenzyl)pyrido[2,3-d]pyrimidin-2,4-diamin,
N⁴-(3,5-Dimethoxybenzyl)-N²-(2-pyridin-4-ylethyl)pyrido[2,3-d]pyrimidin-2,4-diamin,
N⁴-(3,5-Dimethoxybenzyl)-N²-(2-thiophen-2-ylethyl)pyrido[2,3-d]pyrimidin-2,4-diamin,
N⁴- (3, 5-Dimethoxybenzyl) -N²-2-phenethylpyrido[2,3-d]pyrimidin-2,4-diamin,
N⁴-(3, 5-Dimethoxybenzyl)-N²-[2-(3, 5-dimethoxyphenyl)ethyl]pyrido[2,3-d]pyrimidin-2, 4-diamin,
2-(3-{3-[4-(3,4-Dimethoxybenzylamino)pyrido[2,3-d]pyrimidin-2-ylamino]propyl}phenyl)propan-2-ol,
N⁴-(3,4-Dimethoxybenzyl)-N²-[2-(4-fluorphenyl)ethyl]pyrido[2,3-d]pyrimidin-2,4-diamin,
N⁴- (3, 4-Dimethoxybenzyl) -N²-phenethylpyrido [2,3-d]pyrimidin-2,4-diamin oder
N⁴-(3,4-Dimethoxybenzyl)-N²-(3-phenylpropyl)pyrido[2,3-d]pyrimidin-2,4-diamin oder ein pharmazeutisch annehmbares Salz der Verbindung.

5. Pharmazeutische Zusammensetzung, umfassend eine Verbindung der Formel (I) nach einem der Ansprüche 1 bis 4 oder ein pharmazeutisch annehmbares Salz der Verbindung und ein pharmazeutisch annehmbares Vehikel, einen pharmazeutisch annehmbaren Träger oder ein pharmazeutisch annehmbares Verdünnungsmittel.

6. Verwendung einer Verbindung oder eines Salzes gemäß einem der Ansprüche 1 bis 4 bei der Herstellung eines Medikaments zum Behandeln eines PDE 2-vermittelten Zustandes, einer PDE 2-vermittelten Erkrankung oder eines PDE 2-vermittelten Symptoms bei einem Säuger.

7. Verwendung nach Anspruch 6, wobei der Zustand, die Erkrankung oder das Symptom Osteoporose, pulmonaler Hochdruck, Störung der sexuellen Erregung bei Frauen, vermindertes Gedächtnis oder verminderte Wahrnehmung, Thrombozytenaggregation, vaskuläre Angiogenese, Demenz, Krebs, Arrhythmie, Thrombose, Knochenfraktur und/oder -defekt, verzögert konsolidierte oder nicht-konsolidierte Fraktur, Wirbelfusion, Knocheneinwuchs, kraniofaziale Rekonstruktion oder Hypoxie ist.

8. Verwendung nach Anspruch 6, wobei der Zustand Knochenfraktur und/oder -defekt ist.

9. Pharmazeutische Zusammensetzung, umfassend einen PDE 2-Inhibitor gemäß Formel (I) nach Anspruch 1, einen EP₂-selektiven Rezeptoragonisten und ein pharmazeutisch annehmbares Vehikel, einen pharmazeutisch annehmbaren Träger oder ein pharmazeutisch annehmbares Verdünnungsmittel.

10. Zusammensetzung nach Anspruch 9, wobei der PDE 2-Inhibitor N⁴-(3,5-Dimethoxybenzyl)-N²-(2-pyridin-4-yl-ethyl)pyrido[2,3-d]pyrimidin-2,4-diamin, 2-(3-{3-[4-(3,4-Dimethoxybenzylamino)pyrido[2,3-d]pyrimidin-2-ylamino]propyl}phenyl)propan-2-ol, N⁴-(3,4-Dimethoxybenzyl)-N²-(3-phenylpropyl)pyrido[2,3-d]pyrimidin-2,4-diamin oder ein pharmazeutisch annehmbares Salz der Verbindung ist.

11. Zusammensetzung nach Anspruch 9 oder 10, wobei der EP₂-selektive Rezeptoragonist (3-(((4-tert-Butylbenzyl)-(pyridin-3-sulfonyl)amino)methyl)phenoxy)essigsäure oder ein pharmazeutisch annehmbares Salz der Verbindung ist.

12. Verwendung nach einem der Ansprüche 6 bis 8, wobei die Behandlung weiterhin das Verabreichen einer therapeutisch wirksamen Menge eines EP₂-selektiven Rezeptoragonisten oder eine pharmazeutischen Zusammensetzung, umfassend eine Kombination der Verbindung der Formel (I) nach Anspruch 1 und des EP₂-selektiven Rezeptoragonisten, umfasst.

13. Verwendung nach Anspruch 12, wobei der PDE 2-Inhibitor *N*⁴-(3,5-Dimethoxybenzyl)-*N*²-(2-pyridin-4-ylethyl)pyrido[2,3-d]pyrimidin-2,4-diamin, 2-(3-{3-[4-(3,4-Dimethoxybenzylamino)pyrido[2,3-d]pyrimidin-2-ylamino]propyl}phenyl)propan-2-ol, *N*⁴-(3,4-Dimethoxybenzyl)-*N*²-(3-phenylpropyl)pyrido[2,3-d]pyrimidin-2,4-diamin oder ein pharmazeutisch annehmbares Salz der Verbindung ist.

14. Verwendung nach Anspruch 12 oder 13, wobei der EP₂-selektive Rezeptoragonist (3-(((4-tert-Butylbenzyl)-(pyridin-3-sulfonyl)amino)methyl)phenoxy)essigsäure oder ein pharmazeutisch annehmbares Salz der Verbindung ist.

15. Verwendung eines PDE 2-Inhibitors gemäß Formel (I) nach Anspruch 1 oder eines pharmazeutisch annehmbaren Salzes des Inhibitors bei der Herstellung eines Medikaments zur Behandlung von Knochenfraktur und/oder -defekt bei einem Säuger.

16. Verbindung oder Salz gemäß Anspruch 1 zur Verwendung als Medikament.

17. Verbindung oder Salz gemäß Anspruch 1 zur Verwendung als Medikament in der Behandlung eines PDE 2-vermittelten Zustandes, einer PDE 2-vermittelten Erkrankung oder eines PDE 2-vermittelten Symptoms bei einem Säuger.

## Revendications

1. Composé de formule (I) 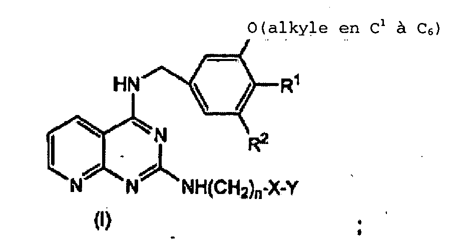 et les sels pharmaceutiquement acceptables de tels composés, formule dans laquelle :
R¹ et R² représentent des atomes d'hydrogène ou des groupes méthoxy, sous réserve que R¹ et R² ne représentent pas l'un et l'autre un atome d'hydrogène ou l'un et l'autre un groupe méthoxy ;
n est égal à 1, 2, 3 ou 4 ;
X représente une liaison ; O ; S ; un groupe C=O; un groupe -N(R)-, dans lequel R représente un atome d'hydrogène ou un groupe -alkyle en C₁ à C₃ ; un groupe -C(OH)-; ou un groupe -SO₂ ; et
Y représente un groupe benzoxazolyle ; benzothiazolyle ; benzofurazannyle ; benzofurannyle ; benzothiadiazolyle ; benzisoxazolyle ; benzisothiazolyle ; benzimidazolyle ; pyridyle ; isatinyle ; oxindolyle ; indazolyle ; indolyle ; phényle ; thiényle ; ou furannyle ; Y étant facultativement substitué indépendamment avec un à trois substituants halogéno ; trifluorométhyle ; méthoxy ; -C(=O)CH₃ ; cyano ; -C(CH₃)₂OH ; -CH(CH₃)OH : -CH(CF₃)OH ; -C(C=)CF₃ ; -SO₂NH₂ ; -C(=O)OCH₃ ; -CH₂COOH ; 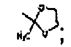 thiazolyle ou oxadiazolyle.

2. Composé suivant la revendication 1, dans lequel X représente une liaison et Y représente un groupe benzofurazannyle ; thiényle ; pyridyle ; ou phényle, le groupe phényle étant facultativement substitué indépendamment avec un ou deux substituants halogéno ; trifluorométhyle ; méthoxy ; -C(=O)CH₃ ; cyano ; -C(CH₃)₂OH ; -CH(CH₃)OH ; -CH(CF₃)OH ; -C(C=O)CF₃; -SO₂NH₂ ; -C(=O)OCH₃; -CH₂COOH ; thiazolyle ; ou oxadiazolyle.

3. Composé suivant la revendication 1 ou 2, dans lequel X représente une liaison, n est égal à 2 ou 3 et Y représente un groupe thiényle ; pyridyle ou phényle, le groupe phényle étant facultativement substitué indépendamment avec un ou deux substituants méthoxy ; halogéno ; -C(CH₃)₂OH; CH(CF₃)OH ; ou -C(C=O)CF₃.

4. *N*²*,N*⁴*-bis*(3,5-diméthoxybenzyl)-pyrido[2,3-d]pyrimidine-2,4-diamine ;
*N*⁴-(3,5-diméthoxybenzyl)-*N*²-(2-pyridine-4-yl-éthyl)-pyrido-[2,3-d]pyrimidine-2,4-diamine;
*N*⁴-(3,5-diméthoxybenzyl)-*N*²-(2-thiophène-2-yl-éthyl)-pyrido[2,3-d]pyrimidine-2,4-diamine;
*N*⁴-(3,5-diméthoxybenzyl) -*N*²-2-phénéthyl-pyrido[2,3-d]-pyrimidine-2,4-diamine ;
*N*⁴-(3,5-diméthoxybenzyl)-*N*²-[2-(3,5-diméthoxyphényl)-éthyl]-pyrido[2,3-d]pyrimidine-2,4-diamine ;
2-(3-{3-[4-(3,4-diméthoxybenzylamino)-pyrido[2,3-d]-pyrimidine-2-ylamino]-propyl}-phényl)-propane-2-ol ;
*N*⁴-(3,4-diméthoxybenzyl)-*N*²-[2-(4-fluorophényl)-éthyl]-pyrido[2,3-d]pyrimidine-2,4-diamine ;
*N*⁴-(3,4-diméthoxybenzyl)-*N*²-phénéthyl-pyrido[2,3-d]pyrimidine-2,4-diamine; ou
*N*⁴-(3,4-diméthoxybenzyl)-*N*²-(3-phényl-propyl)-pyrido[2,3-d]-pyrimidine-2,4-diamine; ou un sel pharmaceutiquement acceptable dudit composé.

5. Composition pharmaceutique comprenant un composé de formule (I) suivant l'une quelconque des revendications 1 à 4, ou un sel pharmaceutiquement acceptable dudit composé, et un véhicule, support ou diluant pharmaceutiquement acceptable.

6. Utilisation d'un composé ou sel suivant l'une quelconque des revendications 1 à 4 dans la production d'un médicament pour le traitement d'une affection, d'une maladie ou d'un symptôme à médiation par la PDE 2 chez un mammifère.

7. Utilisation suivant la revendication 6, dans laquelle ladite affection ou maladie ou ledit symptôme est l'ostéoporose, l'hypertension pulmonaire, un trouble d'éveil sexuel féminin, une diminution de la mémoire ou de la cognition, l'agrégation plaquettaire, l'angiogenèse vasculaire, la démence, le cancer, l'arythmie, la thrombose, une fracture et/ou déficience osseuse, un retard ou défaut de consolidation de fracture, une fusion rachidienne, une croissance en profondeur de tissu osseux, une reconstruction faciale crânienne ou une hypoxie.

8. Utilisation suivant la revendication 6, dans laquelle ladite affection est une fracture et/ou déficience osseuse.

9. Composition pharmaceutique comprenant un inhibiteur de PDE 2 selon la formule (I) de la revendication 1, un agoniste de récepteur sélectif de EP₂ et un véhicule, support ou diluant pharmaceutiquement acceptable.

10. Composition suivant la revendication 9, dans laquelle ledit inhibiteur de PDE 2 est la *N*⁴-(3,5-diméthoxybenzyl)-*N*²-(2-pyridine-4-yl-éthyl)-pyrido[2,3-d]pyrimidine-2,4-diamine ; le 2-(3-{3-[4-(3,4-diméthoxybenzylamino)-pyrido[2,3-d]-pyrimidine-2-ylamino]-propyl}-phényl)propane-2-ol ; la *N*⁴-(3,4-diméthoxybenzyl)-*N*²-(3-phénylpropyl)-pyrido-[2,3-d]pyrimidine-2,4-diamine ; ou un sel pharmaceutiquement acceptable dudit composé.

11. Composition suivant la revendication 9 ou 10, dans laquelle ledit agoniste de récepteur sélectif de EP₂ est l'acide (3-(((4-tertiobutylbenzyl)-(pyridine-3-sulfonyl)-amino)-méthyl)-phénoxy-)acétique, ou un sel pharmaceutiquement acceptable dudit composé.

12. Utilisation suivant l'une quelconque des revendications 6 à 8, pour un traitement qui comprend en outre l'administration audit mammifère d'une quantité thérapeutiquement efficace d'un agoniste de récepteur sélectif de EP₂; ou d'une composition pharmaceutique comprenant une association dudit composé de formule (I) de la revendication 1 et dudit agoniste de récepteur sélectif de EP₂.

13. Utilisation suivant la revendication 12, dans laquelle ledit inhibiteur de PDE 2 est la *N*⁴-(3,5-diméthoxybenzyl)-*N*²-(2-pyridine-4-yl-éthyl)-pyrido[2,3-d]pyrimidine-2,4-diamine ; le 2-(3-{3-[4-(3,4-diméthoxybenzylamino)-pyrido-[2,3-d]pyrimidine-2-ylamino]propyl}-phényl)-propane-2-ol; la *N*⁴- (3,4-diméthoxybenzyl)-*N*²-(3 -phényl-propyl)-pyrido [2,3-d]-pyrimidine-2,4-diamine ; ou un sel pharmaceutiquement acceptable dudit composé.

14. Utilisation suivant la revendication 12 ou 13, dans laquelle ledit agoniste de récepteur sélectif de EP₂ est l'acide (3-(((4-*tertio*butylbenzyl)-(pyridine-3-sulfonyl)-amino)-méthyl)-phénoxy)-acétique, ou un sel pharmaceutiquement acceptable dudit composé.

15. Utilisation d'un inhibiteur de PDE 2 répondant à la formule (I) de la revendication 1, ou d'un sel pharmaceutiquement acceptable dudit inhibiteur, dans la production d'un médicament destiné au traitement d'une fracture osseuse et/ou d'une déficience osseuse chez un mammifère.

16. Composé ou sel suivant la revendication 1, destiné à être utilisé comme médicament.

17. Composé ou sel suivant la revendication 1, destiné à être utilisé comme médicament dans le traitement d'une affection, d'une maladie ou d'un symptôme à médiation par la PDE 2 chez un mammifère.
